(19) Europäisches Patentamt
European Patent Office
Office européen des brevets

(11) **EP 2 820 419 B1**

(12) # EUROPEAN PATENT SPECIFICATION

(45) Date of publication and mention
of the grant of the patent:
**09.12.2020 Bulletin 2020/50**

(51) Int Cl.:
***G01N 33/68*** (2006.01)

(21) Application number: **13755187.5**

(86) International application number:
**PCT/US2013/028005**

(22) Date of filing: **27.02.2013**

(87) International publication number:
**WO 2013/130594 (06.09.2013 Gazette 2013/36)**

(54) **METHODS AND COMPOSITIONS FOR DIAGNOSIS AND PROGNOSIS OF RENAL INJURY AND RENAL FAILURE**

VERFAHREN UND ZUSAMMENSETZUNGEN ZUR DIAGNOSE UND PROGNOSE VON NIERENLÄSION UND NIERENINSUFFIZIENZ

PROCÉDÉS ET COMPOSITIONS DE DIAGNOSTIC ET DE PRONOSTIC DE LÉSION RÉNALE ET D'INSUFFISANCE RÉNALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2012 US 201261603926 P**

(43) Date of publication of application:
**07.01.2015 Bulletin 2015/02**

(73) Proprietor: **Astute Medical, Inc.**
**San Diego, CA 92121 (US)**

(72) Inventors:
• **ANDERBERG, Joseph**
**Encinitas, CA 92024 (US)**
• **GRAY, Jeff**
**Solana Beach, CA 92075 (US)**
• **MCPHERSON, Paul**
**Encinitas, CA 92024 (US)**
• **NAKAMURA, Kevin**
**Cardiff By The Sea, CA 92007 (US)**
• **KAMPF, James, Patrick**
**San Diego, CA 92130 (US)**

(74) Representative: **Wilding, James Roger et al**
**Mathys & Squire LLP**
**The Shard**
**32 London Bridge Street**
**London SE1 9SG (GB)**

(56) References cited:
WO-A1-2012/136548     US-A1- 2007 112 327
US-A1- 2009 148 539     US-A1- 2010 267 041
US-A1- 2013 035 290

• **BERNTSEN ANNIKA ET AL: "Therapeutic dendritic cell vaccination of patients with metastatic renal cell carcinoma - A clinical, phase 1/2 trial", JOURNAL OF IMMUNOTHERAPY, LIPPINCOTT WILLIAMS & WILKINS, HAGERSTOWN, MD, US, vol. 31, no. 8, 1 October 2008 (2008-10-01), pages 771-780, XP009128312, ISSN: 1524-9557, DOI: 10.1097/CJI.0B013E3181833818**
• **JOHANSEN JULIA S: "Studies on serum YKL-40 as a biomarker in diseases with inflammation, tissue remodelling, fibroses and cancer", DANISH MEDICAL BULLETIN, ALMINDELIGE DANSKE LAEGEFORENING, DK, vol. 53, no. 2, 1 May 2006 (2006-05-01), pages 172-209, XP002523827, ISSN: 1603-9629**
• **MADDENS ET AL.: 'Chitinase-like Proteins are Candidate Biomarkers for Sepsis-induced Acute Kidney Injury' MOL CELL PROTEOMICS vol. 11, no. 6, 10 January 2012, pages 1 - 13, XP055163043**

**Description**

.

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830. Renal disease and/or injury may be acute or chronic. Acute and chronic kidney disease are described as follows (from Current Medical Diagnosis & Treatment 2008, 47th Ed, McGraw Hill, New York, pages 785-815): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222:

| Type | Risk Factors |
| --- | --- |
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |

(continued)

| Intrinsic Renal | |
|---|---|
| Acute tubulo interstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| Postrenal | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Type | Risk Factors |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, JAm Soc Nephrol 16: 3365-3370, 2005. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, the RIFLE criteria provide a uniform definition of AKI which has been validated in numerous studies.

More recently, Mehta et al., Crit. Care 11:R31 (doi:10.1186.cc5713), 2007, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4):177-197) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Maddens et al. (2012) inter alia describes urinary biomarkers for septic AKI (Maddens et al. (2012) "Chitinase-like Proteins are Candidate Biomarkers for Sepsis-induced Acute Kidney Injury", Mol Cell Proteomics, vol. 11, no. 6, pages 1-13). Bernsten et al. (2008) inter alia measures serum IL6 and YKL40 in patients with metastatic renal cell carcinoma (Bernsten et al. (2008) "Therapeutic dendritic cell vaccination of patients with metastatic renal cell carcinoma - A clinical, phase ½ trial", Journal of Immunotherapy, vol. 31, no. 8, pages 771-780). Johansen (2006) inter alia describes YKL40 as a biomarker in different diseases (Johansen (2006) "Studies on serum YKL-40 as a biomarker in diseases with inflammation, tissue remodelling, fibroses and cancer", Danish Medical Bulletin, vol. 53, no. 2, (2006-05-01), pages 172-209).

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the disclosure to provide methods and compositions for evaluating renal function in a subject. As described herein, measurement of Chitinase-3-like protein 1 (referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

Chitinase-3-like protein 1 may be used, individually or in panels comprising a plurality of kidney injury markers, for risk stratification (that is, to identify subjects at risk for a future injury to renal function, for future progression to reduced renal function, for future progression to ARF, for future improvement in renal function, etc.); for diagnosis of existing disease (that is, to identify subjects who have suffered an injury to renal function, who have progressed to reduced renal function, who have progressed to ARF, etc.); for monitoring for deterioration or improvement of renal function; and for predicting a future medical outcome, such as improved or worsening renal function, a decreased or increased mortality risk, a decreased or increased risk that a subject will require renal replacement therapy (i.e., hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, etc.

In a first aspect, the present invention relates to a method for determining a subject's risk for progression to ARF , comprising:

performing an assay that generate a detectable signal indicative of the amount of a physiologically relevant concentration of Chitinase-3-like protein 1 on a body fluid sample obtained from the subject to provide an assay result; and

correlating the assay result to the renal status of the subject, wherein said correlation step comprises correlating the assay result to a likelihood of one or more future changes in renal status, said future changes in renal status comprising future acute renal failure (ARF),

and wherein the correlating comprises comparing an assay result in the form of the Chitinase-3-like protein 1 concentration to a predetermined threshold selected to be indicative of the likelihood of future ARF,

wherein an increased likelihood of progression to ARF is assigned to the subject, when the measured concentration is above the threshold relative to a likelihood assigned when the measured concentration is below the threshold,

and wherein the likelihood of future ARF is more or less likely to occur within 48 hours of the time at which the body fluid sample is obtained from the subject.

In a second aspect the present invention relates to a method for evaluating renal status in a subject, comprising:

performing an assay that generates a detectable signal indicative of the amount of a physiologically relevant concentration of Chitinase-3-like protein 1 on a body fluid sample obtained from the subject to provide an assay result; and

correlating the assay result to the renal status of the subject, wherein said correlation step comprises correlating the assay result to a likelihood of one or more future changes in renal status, wherein the future change is future improvement in renal function, and

wherein the correlating comprises comparing an assay result in the form of the Chitinase-3-like protein 1 concentration to a predetermined threshold selected to be indicative of the likelihood of future improvement of renal function,

an increased likelihood of a future improvement in renal function is assigned to the subject when the measured concentration is below the threshold, relative to a likelihood assigned when the measured concentration is above the threshold.

In a third aspect the present invention relates to a use of a measured concentration of Chitinase-3-like protein 1 on a body fluid sample obtained from a subject for assigning a likelihood of one or more future changes in renal status to the subject, selected from the group consisting of a future improvement in renal function and future acute renal failure (ARF), the use comprising

comparing a measured concentration of Chitinase-3-like protein 1 to a threshold value, wherein an increased likelihood of a future improvement in renal function is assigned to the subject when the measured concentration is below the threshold, relative to a likelihood assigned when the measured concentration is above the threshold, or

wherein an increased likelihood of progression to ARF is assigned to the subject, when the measured concentration is above the threshold relative to a likelihood assigned when the measured concentration is below the threshold,

and wherein the likelihood of future ARF is more or less likely to occur within 48 hours of the time at which the body fluid sample is obtained from the subject.

Also described are methods for evaluating renal status in a subject. These methods comprise performing an assay method that is configured to detect Chitinase-3-like protein 1, the result of which is then correlated to the renal status of the subject. This correlation to renal status may include correlating the assay result to one or more of risk stratification, diagnosis, prognosis, staging, classifying and monitoring of the subject as described herein. Thus, the methods utilize one or more kidney injury markers disclosed herein for the evaluation of renal injury.

In certain aspects, the methods for evaluating renal status described herein are methods for risk stratification of the subject; that is, assigning a likelihood of one or more future changes in renal status to the subject. In these embodiments, the Chitinase-3-like protein 1 assay result is correlated to one or more such future changes. The following are preferred risk stratification embodiments.

In preferred risk stratification aspects, , these methods comprise determining a subject's risk for a future injury to renal function, and the Chitinase-3-like protein 1 assay result is correlated to a likelihood of such a future injury to renal function. For example, the measured concentration may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification aspects, these methods comprise determining a subject's risk for future reduced renal function, and the Chitinase-3-like protein 1 assay result is correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification aspects, these methods comprise determining a subject's likelihood for a future improvement in renal function, and the Chitinase-3-like protein 1 assay resultis correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification aspects, these methods comprise determining a subject's risk for progression to ARF, and the Chitinase-3-like protein 1 assay result is correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification aspects, these methods comprise determining a subject's outcome risk, and the Chitinase-3-like protein 1 assay result is correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is below the threshold, relative to a likelihood assigned when the measured concentration is above the threshold.

In such risk stratification aspects, preferably the likelihood or risk assigned is that an event of interest is more or less likely to occur within 180 days of the time at which the body fluid sample is obtained from the subject. In particularly preferred

aspects, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 18 hours, 12 hours, or less. A risk at 0 hours of the time at which the body fluid sample is obtained from the subject is equivalent to diagnosis of a current condition.

In preferred risk stratification aspects , the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred

aspects, a subject is chosen for risk stratification based on an existing diagnosis of injury to renal function, reduced renal function, or ARF.

In other aspects , the methods for evaluating renal status described herein are methods for diagnosing a renal injury in the subject; that is, assessing whether or not a subject has suffered from an injury to renal function, reduced renal function, or ARF. In these embodiments, the Chitinase-3-like protein 1 assay result is correlated to the occurrence or nonoccurrence of a change in renal status. The following are preferred diagnostic aspects.

In preferred diagnostic aspects , these methods comprise diagnosing the occurrence or nonoccurrence of an injury to renal function, and the Chitinase-3-like protein 1 assay result is correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic aspects, these methods comprise diagnosing the occurrence or nonoccurrence of reduced renal function, and the Chitinase-3-like protein 1 assay result is correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold).

For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic aspects, these methods comprise diagnosing the occurrence or nonoccurrence of ARF, and the Chitinase-3-like protein 1 assay result is correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic aspects, these methods comprise diagnosing a subject as being in need of renal replacement therapy, and the Chitinase-3-like protein 1 assay result is correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic aspects, these methods comprise diagnosing a subject as being in need of renal transplantation, and the Chitinase-3-like protein 1 assay result is correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other aspects the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the Chitinase-3-like protein 1 assay result is correlated to the occurrence or nonoccurrence of a change in renal status. The following are preferred monitoring aspects.

In preferred monitoring aspects, these methods comprise monitoring renal status in a subject suffering from an injury to renal function, and the assay result(s) is/are correlated to the occurrence or nonoccurrence of a change in renal status in the subject. For example, the measured Chitinase-3-like protein 1 concentration may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring aspects, these methods comprise monitoring renal status in a subject suffering

8

from reduced renal function, and the Chitinase-3-like protein 1 assay result is correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring aspects, these methods comprise monitoring renal status in a subject suffering from acute renal failure, and the Chitinase-3-like protein 1 assay result is correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring aspects, these methods comprise monitoring renal status in a subject at risk of an injury to renal function due to the pre-existence of one or more known risk factors for prerenal, intrinsic renal, or postrenal ARF, and the Chitinase-3-like protein 1 assay result is correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other aspects, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the Chitinase-3-like protein 1 assay result is correlated to a particular class and/or subclass. The following are preferred classification aspects.

In preferred classification aspects, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the Chitinase-3-like protein 1 assay result is correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, etc.), by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that a Chitinase-3-like protein 1 assay result must be compared to corresponding individual thresholds. Methods for combining assay results can comprise the use of multivariate logistical regression, loglinear modeling, neural network analysis, n-of-m analysis, decision tree analysis, calculating ratios of markers, etc. This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma. In the case of those kidney injury markers which are membrane proteins as described hereinafter, preferred assays detect soluble forms thereof.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score, risk scores of Thakar et al. (J. Am. Soc. Nephrol. 16: 162-68, 2005), Mehran et al. (J. Am. Coll. Cardiol. 44: 1393-99, 2004), Wijeysundera et al. (JAMA 297: 1801-9, 2007), Goldstein and Chawla (Clin. J. Am. Soc. Nephrol. 5: 943-49, 2010), or Chawla et al. (Kidney Intl. 68: 2274-80, 2005)), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, devices and kits for performing the methods described herein are also disclosed. Suitable kits comprise reagents sufficient for performing an assay for at least Chitinase-3-like protein 1, together with instructions for performing the described threshold comparison(s).

In certain aspects, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, etc.) as well as molecules that may be indirectly detected by production of a detectable reaction product (e.g., enzymes such as horseradish peroxidase, alkaline phosphatase, etc.) or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, etc. In certain of these methods, the solid phase antibody is coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

## DETAILED DESCRIPTION OF THE INVENTION

The present disclosure relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of Chitinase-3-like protein 1 or one or more markers related thereto is correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0.5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

Chitinase-3-like protein 1 precursor (Swiss-Prot P36222 (SEQ ID NO: 1)):

```
        10         20         30         40         50         60
MGVKASQTGF VVLVLLQCCS AYKLVCYYTS WSQYREGDGS CFPDALDRFL CTHIIYSFAN

        70         80         90        100        110        120
ISNDHIDTWE WNDVTLYGML NTLKNRNPNL KTLLSVGGWN FGSQRFSKIA SNTQSRRTFI

       130        140        150        160        170        180
KSVPPFLRTH GFDGLDLAWL YPGRRDKQHF TTLIKEMKAE FIKEAQPGKK QLLLSAALSA

       190        200        210        220        230        240
GKVTIDSSYD IAKISQHLDF ISIMTYDFHG AWRGTTGHHS PLFRGQEDAS PDRFSNTDYA

       250        260        270        280        290        300
VGYMLRLGAP ASKLVMGIPT FGRSFTLASS ETGVGAPISG PGIPGRFTKE AGTLAYYEIC

       310        320        330        340        350        360
DFLRGATVHR ILGQQVPYAT KGNQWVGYDD QESVKSKVQY LKDRQLAGAM VWALDLDDFQ

       370        380
GSFCGQDLRF PLTNAIKDAL AAT
```

The following domains have been identified in Chitinase-3-like protein 1:

| Residues | Length | Domain ID |
|---|---|---|
| 1-21 | 21 | Signal peptide |
| 22-383 | 362 | Chitinase-3-like protein 1 |

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, etc.

In this regard, the skilled artisan will understand that the signals obtained from an immunoassay are a direct result of complexes formed between one or more antibodies and the target biomolecule (*i.e.*, the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc*.) and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker disclosed herein, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and *The Immunoassay Handbook,* David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, *etc.*) as well as molecules that may be indirectly detected by production of a detectable reaction product (e.g., enzymes such as horseradish peroxidase, alkaline phosphatase, *etc.*) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homo functional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, kits for the analysis of the described kidney injury markers are disclosed. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker disclosed herein. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ $M^{-1}$, and preferably between about $10^8$ $M^{-1}$ to about $10^9$ $M^{-1}$, about $10^9$ $M^{-1}$ to about $10^{10}$ $M^{-1}$, or about $10^{10}$ $M^{-1}$ to about $10^{12}$ $M^{-1}$ .

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and non conformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g*, Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s). These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, O00622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (O00206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, O00458); Isovaleryl-CoA dehydrogenase (IVD, P26440); I-TAC/CXCL11 (014625); Keratin 19 (P08727); Kim-1 (Hepatitis A virus cellular receptor 1, O43656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (O60356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s).

Other clinical indicia which may be combined with the kidney injury marker assay result(s) includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (*e.g.*, blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 m$^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers disclosed herein may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments and are exemplary.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria

males and females 18 years of age or older;

undergoing cardiovascular surgery;

Toronto/Ottawa Predictive Risk Index for Renal Replacement risk score of at least 2 (Wijeysundera et al., JAMA 297: 1801-9, 2007); and

able and willing to provide written informed consent for study participation and to comply with all study procedures.

Exclusion Criteria

known pregnancy;

previous renal transplantation;

acutely worsening renal function prior to enrollment (e.g., any category of RIFLE criteria);

already receiving dialysis (either acute or chronic) or in imminent need of dialysis at enrollment;

currently enrolled in another clinical study or expected to be enrolled in another clinical study within 7 days of cardiac surgery that involves drug infusion or a therapeutic intervention for AKI;

known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or heplock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 1900 adults expected to be in the ICU for at least 48 hours will be enrolled. To be enrolled in the study, each patient must meet all of the following inclusion criteria and none of the following exclusion criteria:

Inclusion Criteria

males and females 18 years of age or older;

Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment;

contrast media exposure within 24 hours of enrollment;

increased Intra-Abdominal Pressure with acute decompensated heart failure; and

severe trauma as the primary reason for ICU admission and likely to be hospitalized in the ICU for 48 hours after enrollment;

Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment;

Study population 4: approximately 1000 patients that are 21 years of age or older, within 24 hours of being admitted into the ICU, expected to have an indwelling urinary catheter for at least 48 hours after enrollment, and have at least one of the following acute conditions within 24 hours prior to enrollment:
(i) respiratory SOFA score of $\geq$ 2 (PaO2/FiO2 <300), (ii) cardiovascular SOFA score of $\geq$ 1 (MAP < 70 mm Hg and/or any vasopressor required).

Exclusion Criteria

known pregnancy;

institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets any of the following:

(i) active bleeding with an anticipated need for > 4 units PRBC in a day;

(ii) hemoglobin < 7 g/dL;

(iii) any other condition that in the physician's opinion would contraindicate drawing serial blood samples for clinical study purposes;

meets only the SBP < 90 mmHg inclusion criterion set forth above, and does not have shock in the attending physician's or principal investigator's opinion;

After obtaining informed consent, an EDTA anti-coagulated blood sample (10 mL) and a urine sample (25-50 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), 36 ($\pm$ 2), 48 ($\pm$ 2), 60 ($\pm$ 2), 72 ($\pm$ 2), and 84 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Results reported below for Chitinase-3-like protein 1 are in ng/mL.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject was hospitalized. Markers were each measured by standard immunoassay methods using commercially available assay reagents in the urine samples and the plasma component of the blood samples collected.

Two cohorts were defined to represent a "diseased" and a "normal" population. While these terms were used for convenience, "diseased" and "normal" simply represent two cohorts for comparison (say RIFLE 0 vs RIFLE R, I and F; RIFLE 0 vs RIFLE R; RIFLE 0 and R vs RIFLE I and F; etc.). The time "prior max stage" represents the time at which a sample was collected, relative to the time a particular patient reaches the lowest disease stage as defined for that cohort, binned into three groups which were +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) was determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may include patients adjudicated to stage R, I, or F on the basis of urine output; for those patients adjudicated to stage R, I, or F on the basis of urine output alone, the stage 0 cohort may include patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage was used.

The ability to distinguish cohort 1 from Cohort 2 was determined using ROC analysis. SE was the standard error of the AUC, n was the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test. An AUC < 0.5 was indicative of a negative going marker for the comparison, and an AUC > 0.5 was indicative of a positive going marker for the comparison.

Various threshold (or "cutoff") concentrations were selected, and the associated sensitivity and specificity for distinguishing cohort 1 from cohort 2 were determined. OR was the odds ratio calculated for the particular cutoff concentration, and 95% CI was the confidence interval for the odds ratio.

Table 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

**Chitinase-3-like protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.01 | 6.37 | 1.01 | 6.04 | 1.01 | 1.13 |
| Average | 10.4 | 32.4 | 10.4 | 24.3 | 10.4 | 2.52 |
| Stdev | 31.8 | 57.3 | 31.8 | 34.6 | 31.8 | 2.50 |
| p(t-test) | | 0.044 | | 0.12 | | 0.59 |
| Min | 6.15E-5 | 0.000108 | 6.15E-5 | 0.118 | 6.15E-5 | 0.404 |
| Max | 195 | 237 | 195 | 117 | 195 | 6.26 |
| n (Samp) | 47 | 22 | 47 | 20 | 47 | 5 |
| n (Patient) | 22 | 22 | 22 | 20 | 22 | 5 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.24 | 4.15 | 1.24 | 12.9 | 1.24 | 3.24 |
| Average | 18.0 | 42.9 | 18.0 | 31.7 | 18.0 | 7.77 |
| Stdev | 45.0 | 56.7 | 45.0 | 36.3 | 45.0 | 12.3 |
| p(t-test) | | 0.24 | | 0.40 | | 0.61 |
| Min | 6.15E-5 | 1.03 | 6.15E-5 | 1.05 | 6.15E-5 | 0.966 |
| Max | 237 | 119 | 237 | 96.9 | 237 | 29.6 |
| n (Samp) | 98 | 5 | 98 | 8 | 98 | 5 |
| n (Patient) | 47 | 5 | 47 | 8 | 47 | 5 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.21 | 9.09 | 1.21 | 4.83 | 1.21 | 0.952 |
| Average | 12.4 | 36.0 | 12.4 | 24.4 | 12.4 | 32.4 |
| Stdev | 32.9 | 59.1 | 32.9 | 36.3 | 32.9 | 87.3 |
| p(t-test) | | 0.040 | | 0.19 | | 0.24 |
| Min | 6.15E-5 | 0.000108 | 6.15E-5 | 0.118 | 6.15E-5 | 0.404 |
| Max | 195 | 237 | 195 | 117 | 195 | 248 |
| n (Samp) | 47 | 20 | 47 | 20 | 47 | 8 |
| n (Patient) | 22 | 20 | 22 | 20 | 22 | 8 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | 0.71 | 0.74 | 0.72 | 0.77 | 0.68 | 0.56 | 0.62 | 0.51 |
| SE | 0.067 | 0.13 | 0.071 | 0.072 | 0.10 | 0.075 | 0.14 | 0.14 | 0.11 |
| p | 2.3E-4 | 0.11 | 8.7E-4 | 0.0018 | 0.0070 | 0.018 | 0.68 | 0.37 | 0.94 |
| nCohort 1 | 47 | 98 | 47 | 47 | 98 | 47 | 47 | 98 | 47 |
| nCohort 2 | 22 | 5 | 20 | 20 | 8 | 20 | 5 | 5 | 8 |
| Cutoff 1 | 1.76 | 1.49 | 2.85 | 2.83 | 4.26 | 1.21 | 0.819 | 1.08 | 0.637 |
| Sens 1 | 73% | 80% | 70% | 70% | 75% | 70% | 80% | 80% | 75% |
| Spec 1 | 66% | 54% | 66% | 74% | 70% | 51% | 45% | 47% | 38% |
| Cutoff 2 | 1.21 | 1.49 | 1.76 | 0.966 | 3.73 | 0.966 | 0.819 | 1.08 | 0.585 |
| Sens 2 | 82% | 80% | 80% | 80% | 88% | 80% | 80% | 80% | 88% |
| Spec 2 | 57% | 54% | 60% | 49% | 68% | 45% | 45% | 47% | 36% |
| Cutoff 3 | 1.01 | 1.01 | 1.01 | 0.637 | 1.01 | 0.637 | 0.390 | 0.947 | 0.303 |
| Sens 3 | 91% | 100% | 90% | 90% | 100% | 90% | 100% | 100% | 100% |
| Spec 3 | 51% | 45% | 47% | 40% | 45% | 38% | 23% | 43% | 21% |
| Cutoff 4 | 1.88 | 4.26 | 3.85 | 1.88 | 4.26 | 3.85 | 1.88 | 4.26 | 3.85 |
| Sens 4 | 68% | 40% | 65% | 70% | 75% | 55% | 40% | 20% | 25% |
| Spec 4 | 70% | 70% | 70% | 70% | 70% | 70% | 70% | 70% | 70% |
| Cutoff 5 | 5.53 | 13.3 | 7.32 | 5.53 | 13.3 | 7.32 | 5.53 | 13.3 | 7.32 |
| Sens 5 | 50% | 40% | 60% | 50% | 50% | 40% | 20% | 20% | 12% |
| Spec 5 | 81% | 81% | 81% | 81% | 81% | 81% | 81% | 81% | 81% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 | 17.8 | 62.5 | 62.5 | 17.8 | 62.5 | 62.5 | 17.8 | 62.5 | 62.5 |
| Sens 6 | 32% | 40% | 20% | 35% | 25% | 15% | 0% | 0% | 12% |
| Spec 6 | 91% | 91% | 91% | 91% | 91% | 91% | 91% | 91% | 91% |
| OR Quart 2 | 6.7 | >1.0 | 4.6 | 2.2 | >1.0 | 2.9 | 1.0 | >2.1 | 4.8 |
| | 0.10 | <1.0 | 0.20 | 0.42 | <1.0 | 0.25 | 1.0 | <0.56 | 0.19 |
| p Value | 0.69 | >0.059 | 0.46 | 0.34 | >0.059 | 0.48 | 0.056 | >0.18 | 0.46 |
| 95% CI of OR Quart2 | 65 | na | 47 | 14 | na | 18 | 18 | na | 50 |
| OR Quart 3 | 6.7 | >2.1 | 8.2 | 2.9 | >3.4 | 2.9 | 1.0 | >2.1 | 0.92 |
| | 0.10 | <0.56 | 0.068 | 0.25 | <0.30 | 0.25 | 1.0 | <0.56 | 0.96 |
| p Value | 0.69 | >0.18 | 0.86 | 0.48 | >0.33 | 0.48 | 0.056 | >0.18 | 0.052 |
| 95% CI of OR Quart3 | 65 | na | 78 | 18 | na | 18 | 18 | na | 16 |
| OR Quart 4 | 25 | >2.1 | 17 | 7.9 | >4.5 | 6.2 | 2.2 | >1.0 | 2.0 |
| | 0.0046 | <0.56 | 0.013 | 0.022 | <0.19 | 0.042 | 0.55 | <1.0 | 0.59 |
| p Value | 2.7 | >0.18 | 1.8 | 1.4 | >0.47 | 1.1 | 0.17 | >0.059 | 0.16 |
| 95% CI of OR Quart4 | 230 | na | 160 | 46 | na | 36 | 28 | na | 25 |

Table 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

**Chitinase-3-like protein 1**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 1.20 | 6.37 | 1.20 | 7.05 |
| Average | 12.7 | 33.1 | 12.7 | 32.7 |
| Stdev | 36.7 | 50.0 | 36.7 | 54.3 |
| p(t-test) | | 0.045 | | 0.047 |
| Min | 6.15E-5 | 0.000108 | 6.15E-5 | 0.341 |
| Max | 237 | 166 | 237 | 206 |
| n (Samp) | 92 | 18 | 92 | 20 |
| n (Patient) | 44 | 18 | 44 | 20 |

| sCr only | 0hr prior to AKI stage | | 24hr prior to AKI stage | |
|---|---|---|---|---|
| | Cohort 1 | Cohort 2 | Cohort 1 | Cohort 2 |
| Median | 1.52 | 59.6 | 1.52 | 88.6 |
| Average | 18.3 | 59.6 | 18.3 | 64.3 |
| Stdev | 46.2 | 84.3 | 46.2 | 49.5 |
| p(t-test) | | 0.22 | | 0.091 |
| Min | 6.15E-5 | 0.000108 | 6.15E-5 | 7.33 |
| Max | 248 | 119 | 248 | 96.9 |

| sCr only | 0hr prior to AKI stage | | 24hr prior to AKI stage | |
|---|---|---|---|---|
| | Cohort 1 | Cohort 2 | Cohort 1 | Cohort 2 |
| n (Samp) | 124 | 2 | 124 | 3 |
| n (Patient) | 60 | 2 | 60 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | 1.16 | 8.47 | 1.16 | 6.77 |
| Average | 13.8 | 35.0 | 13.8 | 34.0 |
| Stdev | 38.2 | 50.9 | 38.2 | 55.5 |
| p(t-test) | | 0.051 | | 0.060 |
| Min | 6.15E-5 | 0.0514 | 6.15E-5 | 0.341 |
| Max | 237 | 166 | 237 | 206 |
| n (Samp) | 84 | 17 | 84 | 19 |
| n (Patient) | 40 | 17 | 40 | 19 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.64 | 0.50 | 0.67 | 0.70 | 0.88 | 0.70 |
| SE | 0.076 | 0.21 | 0.077 | 0.070 | 0.13 | 0.072 |
| p | 0.070 | 0.98 | 0.027 | 0.0033 | 0.0040 | 0.0062 |
| nCohort 1 | 92 | 124 | 84 | 92 | 124 | 84 |
| nCohort 2 | 18 | 2 | 17 | 20 | 3 | 19 |
| Cutoff 1 | 0.760 | 6.15E-5 | 1.26 | 1.63 | 7.32 | 1.26 |
| Sens 1 | 72% | 100% | 71% | 70% | 100% | 74% |
| Spec 1 | 37% | 3% | 55% | 59% | 75% | 55% |
| Cutoff 2 | 0.689 | 6.15E-5 | 0.733 | 1.07 | 7.32 | 0.947 |
| Sens 2 | 83% | 100% | 82% | 80% | 100% | 84% |
| Spec 2 | 35% | 3% | 38% | 47% | 75% | 43% |
| Cutoff 3 | 0.000108 | 6.15E-5 | 0.0888 | 0.875 | 7.32 | 0.637 |
| Sens 3 | 94% | 100% | 94% | 90% | 100% | 95% |
| Spec 3 | 7% | 3% | 11% | 39% | 75% | 36% |
| Cutoff 4 | 3.76 | 5.32 | 3.86 | 3.76 | 5.32 | 3.86 |
| Sens 4 | 56% | 50% | 59% | 55% | 100% | 53% |
| Spec 4 | 71% | 70% | 70% | 71% | 70% | 70% |
| Cutoff 5 | 7.04 | 13.7 | 8.95 | 7.04 | 13.7 | 8.95 |
| Sens 5 | 50% | 50% | 47% | 50% | 67% | 47% |
| Spec 5 | 80% | 81% | 81% | 80% | 81% | 81% |
| Cutoff 6 | 33.1 | 62.5 | 36.4 | 33.1 | 62.5 | 36.4 |
| Sens 6 | 28% | 50% | 29% | 25% | 67% | 26% |
| Spec 6 | 90% | 90% | 90% | 90% | 90% | 90% |
| OR Quart 2 | 0.96 | 0 | 1.6 | 5.9 | >0 | 4.4 |
| p Value | 0.96 | na | 0.64 | 0.12 | <na | 0.20 |
| 95% CI of | 0.18 | na | 0.24 | 0.64 | >na | 0.45 |
| OR Quart2 | 5.2 | na | 10 | 54 | na | 42 |

| | 0hr prior to AKI stage | | | 24hr prior to AKI stage | | |
|---|---|---|---|---|---|---|
| | sCr or UO | sCr only | UO only | sCr or UO | sCr only | UO only |
| OR Quart 3 | 1.0 | 0 | 1.6 | 4.5 | >1.0 | 5.7 |
| p Value | 1.0 | na | 0.64 | 0.19 | <1.0 | 0.12 |
| 95% CI of | 0.18 | na | 0.24 | 0.47 | >0.060 | 0.62 |
| OR Quart3 | 5.5 | na | 10 | 43 | na | 53 |
| OR Quart 4 | 3.8 | 1.0 | 6.1 | 15 | >2.1 | 13 |
| p Value | 0.070 | 0.98 | 0.032 | 0.013 | <0.56 | 0.021 |
| 95% CI of | 0.90 | 0.062 | 1.2 | 1.8 | >0.18 | 1.5 |
| OR Quart4 | 16 | 17 | 32 | 130 | na | 110 |

Table 3: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

**Chitinase-3-like protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.70 | 63.0 | 1.70 | 25.2 | 1.70 | 29.6 |
| Average | 14.1 | 80.2 | 14.1 | 65.6 | 14.1 | 93.4 |
| Stdev | 42.5 | 83.5 | 42.5 | 86.0 | 42.5 | 135 |
| p(t-test) | | 0.0075 | | 0.036 | | 0.033 |
| Min | 0.0888 | 2.04 | 0.0888 | 0.966 | 0.0888 | 2.04 |
| Max | 195 | 248 | 195 | 248 | 195 | 248 |
| n (Samp) | 22 | 8 | 22 | 8 | 22 | 3 |
| n (Patient) | 22 | 8 | 22 | 8 | 22 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.19 | 63.0 | 2.19 | 25.2 | 2.19 | 29.6 |
| Average | 18.1 | 80.2 | 18.1 | 65.6 | 18.1 | 93.4 |
| Stdev | 43.8 | 83.5 | 43.8 | 86.0 | 43.8 | 135 |
| p(t-test) | | 0.012 | | 0.054 | | 0.045 |
| Min | 0.0888 | 2.04 | 0.0888 | 0.966 | 0.0888 | 2.04 |
| Max | 195 | 248 | 195 | 248 | 195 | 248 |
| n (Samp) | 22 | 8 | 22 | 8 | 22 | 3 |
| n (Patient) | 22 | 8 | 22 | 8 | 22 | 3 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.90 | nd | 0.86 | 0.82 | nd | 0.78 | 0.85 | nd | 0.79 |
| SE | 0.078 | nd | 0.089 | 0.098 | nd | 0.11 | 0.15 | nd | 0.16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 3.1E-7 | nd | 6.3E-5 | 9.0E-4 | nd | 0.0068 | 0.017 | nd | 0.079 |
| nCohort 1 | 22 | nd | 22 | 22 | nd | 22 | 22 | nd | 22 |
| nCohort 2 | 8 | nd | 8 | 8 | nd | 8 | 3 | nd | 3 |
| Cutoff 1 | 17.8 | nd | 18.7 | 7.32 | nd | 7.32 | 1.88 | nd | 1.76 |
| Sens 1 | 75% | nd | 75% | 75% | nd | 75% | 100% | nd | 100% |
| Spec 1 | 91% | nd | 86% | 86% | nd | 77% | 64% | nd | 50% |
| Cutoff 2 | 7.32 | nd | 7.32 | 1.88 | nd | 1.76 | 1.88 | nd | 1.76 |
| Sens 2 | 88% | nd | 88% | 88% | nd | 88% | 100% | nd | 100% |
| Spec 2 | 86% | nd | 77% | 64% | nd | 50% | 64% | nd | 50% |
| Cutoff 3 | 1.88 | nd | 1.76 | 0.942 | nd | 0.942 | 1.88 | nd | 1.76 |
| Sens 3 | 100% | nd | 100% | 100% | nd | 100% | 100% | nd | 100% |
| Spec 3 | 64% | nd | 50% | 36% | nd | 36% | 64% | nd | 50% |
| Cutoff 4 | 2.83 | nd | 5.32 | 2.83 | nd | 5.32 | 2.83 | nd | 5.32 |
| Sens 4 | 88% | nd | 88% | 75% | nd | 75% | 67% | nd | 67% |
| Spec 4 | 73% | nd | 73% | 73% | nd | 73% | 73% | nd | 73% |
| Cutoff 5 | 3.85 | nd | 17.8 | 3.85 | nd | 17.8 | 3.85 | nd | 17.8 |
| Sens 5 | 88% | nd | 75% | 75% | nd | 62% | 67% | nd | 67% |
| Spec 5 | 82% | nd | 82% | 82% | nd | 82% | 82% | nd | 82% |
| Cutoff 6 | 17.8 | nd | 62.5 | 17.8 | nd | 62.5 | 17.8 | nd | 62.5 |
| Sens 6 | 75% | nd | 50% | 62% | nd | 38% | 67% | nd | 33% |
| Spec 6 | 91% | nd | 91% | 91% | nd | 91% | 91% | nd | 91% |
| OR Quart 2 | >1.0 | nd | >1.0 | >1.0 | nd | >2.3 | >0 | nd | >1.2 |
| | <1.0 | nd | <1.0 | <1.0 | nd | <0.53 | <na | nd | <0.91 |
| p Value | >0.052 | nd | >0.052 | >0.052 | nd | >0.17 | >na | nd | >0.059 |
| 95% CI of OR Quart2 | na | nd | na | na | nd | na | na | nd | na |
| OR Quart 3 | >1.2 | nd | >2.8 | >2.8 | nd | >1.2 | >1.2 | nd | >0 |
| | <0.92 | nd | <0.45 | <0.45 | nd | <0.92 | <0.91 | nd | <na |
| p Value | >0.059 | nd | >0.20 | >0.20 | nd | >0.059 | >0.059 | nd | >na |
| 95% CI of OR Quart3 | na | nd | na | na | nd | na | na | nd | na |
| OR Quart 4 | >21 | nd | >12 | >12 | nd | >12 | >2.4 | nd | >2.4 |
| | <0.024 | nd | <0.058 | <0.058 | nd | <0.058 | <0.52 | nd | <0.52 |
| p Value | >1.5 | nd | >0.92 | >0.92 | nd | >0.92 | >0.16 | nd | >0.16 |
| 95% CI of OR Quart4 | na | nd | na | na | nd | na | na | nd | na |

Table 4: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

**Chitinase-3-like protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 285 | 347 | 285 | 578 | 285 | 321 |
| Average | 582 | 760 | 582 | 1330 | 582 | 378 |
| Stdev | 1010 | 1260 | 1010 | 1730 | 1010 | 247 |
| p(t-test) | | 0.53 | | 0.031 | | 0.66 |
| Min | 18.3 | 82.9 | 18.3 | 16.8 | 18.3 | 159 |
| Max | 6000 | 5010 | 6000 | 6000 | 6000 | 783 |
| n (Samp) | 50 | 21 | 50 | 18 | 50 | 5 |
| n (Patient) | 25 | 21 | 25 | 18 | 25 | 5 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 282 | 803 | 282 | 563 | 282 | 594 |
| Average | 602 | 1690 | 602 | 1000 | 602 | 698 |
| Stdev | 1080 | 2290 | 1080 | 1220 | 1080 | 705 |
| p(t-test) | | 0.062 | | 0.35 | | 0.85 |
| Min | 12.1 | 164 | 12.1 | 164 | 12.1 | 104 |
| Max | 6000 | 5010 | 6000 | 3470 | 6000 | 1850 |
| n (Samp) | 99 | 4 | 99 | 7 | 99 | 5 |
| n (Patient) | 49 | 4 | 49 | 7 | 49 | 5 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 288 | 383 | 288 | 546 | 288 | 321 |
| Average | 588 | 804 | 588 | 1450 | 588 | 1100 |
| Stdev | 995 | 1280 | 995 | 1770 | 995 | 2160 |
| p(t-test) | | 0.45 | | 0.012 | | 0.29 |
| Min | 18.3 | 82.9 | 18.3 | 16.8 | 18.3 | 159 |
| Max | 6000 | 5010 | 6000 | 6000 | 6000 | 6000 |
| n (Samp) | 51 | 20 | 51 | 19 | 51 | 7 |
| n (Patient) | 26 | 20 | 26 | 19 | 26 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.63 | 0.56 | 0.66 | 0.64 | 0.67 | 0.55 | 0.61 | 0.55 |
| SE | 0.076 | 0.15 | 0.077 | 0.079 | 0.12 | 0.076 | 0.14 | 0.14 | 0.12 |
| p | 0.50 | 0.40 | 0.42 | 0.048 | 0.23 | 0.024 | 0.71 | 0.44 | 0.67 |
| nCohort 1 | 50 | 99 | 51 | 50 | 99 | 51 | 50 | 99 | 51 |
| nCohort 2 | 21 | 4 | 20 | 18 | 7 | 19 | 5 | 5 | 7 |
| Cutoff 1 | 178 | 178 | 222 | 252 | 211 | 303 | 197 | 144 | 252 |
| Sens 1 | 71% | 75% | 70% | 72% | 71% | 74% | 80% | 80% | 71% |
| Spec 1 | 36% | 33% | 43% | 48% | 41% | 55% | 42% | 27% | 49% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 162 | 162 | 168 | 178 | 178 | 213 | 197 | 144 | 197 |
| Sens 2 | 81% | 100% | 80% | 83% | 86% | 84% | 80% | 80% | 86% |
| Spec 2 | 28% | 28% | 27% | 36% | 33% | 41% | 42% | 27% | 39% |
| Cutoff 3 | 104 | 162 | 104 | 76.7 | 162 | 76.7 | 144 | 90.5 | 144 |
| Sens 3 | 90% | 100% | 90% | 94% | 100% | 95% | 100% | 100% | 100% |
| Spec 3 | 16% | 28% | 14% | 16% | 28% | 14% | 26% | 18% | 24% |
| Cutoff 4 | 466 | 445 | 485 | 466 | 445 | 485 | 466 | 445 | 485 |
| Sens 4 | 29% | 50% | 30% | 56% | 57% | 53% | 20% | 60% | 14% |
| Spec 4 | 70% | 71% | 71% | 70% | 71% | 71% | 70% | 71% | 71% |
| Cutoff 5 | 675 | 675 | 675 | 675 | 675 | 675 | 675 | 675 | 675 |
| Sens 5 | 24% | 50% | 25% | 39% | 29% | 42% | 20% | 40% | 14% |
| Spec 5 | 80% | 81% | 80% | 80% | 81% | 80% | 80% | 81% | 80% |
| Cutoff 6 | 1290 | 1290 | 1290 | 1290 | 1290 | 1290 | 1290 | 1290 | 1290 |
| Sens 6 | 14% | 50% | 15% | 33% | 29% | 37% | 0% | 20% | 14% |
| Spec 6 | 90% | 91% | 90% | 90% | 91% | 90% | 90% | 91% | 90% |
| OR Quart 2 | 1.2 | >2.1 | 0.65 | 1.0 | >3.2 | 2.1 | >2.2 | 1.0 | 2.0 |
| | 0.77 | <0.56 | 0.61 | 1.0 | <0.32 | 0.42 | <0.55 | 1.0 | 0.59 |
| p Value | 0.27 | >0.18 | 0.12 | 0.17 | >0.32 | 0.34 | >0.17 | 0.059 | 0.16 |
| 95% CI of OR Quart2 | 5.7 | na | 3.5 | 5.8 | na | 14 | na | 17 | 25 |
| OR Quart 3 | 1.6 | >0 | 2.6 | 1.9 | >0 | 3.1 | >2.2 | 0 | 3.5 |
| | 0.52 | <na | 0.20 | 0.42 | <na | 0.22 | <0.55 | na | 0.30 |
| p Value | 0.37 | >na | 0.61 | 0.38 | >na | 0.51 | >0.17 | na | 0.32 |
| 95% CI of OR Quart3 | 7.2 | na | 11 | 9.9 | na | 19 | na | na | 39 |
| OR Quart 4 | 1.6 | >2.1 | 1.2 | 3.3 | >4.5 | 6.0 | >1.0 | 3.3 | 0.93 |
| | 0.52 | <0.56 | 0.77 | 0.14 | <0.19 | 0.044 | <1.0 | 0.32 | 0.96 |
| p Value | 0.37 | >0.18 | 0.27 | 0.67 | >0.47 | 1.0 | >0.056 | 0.32 | 0.053 |
| 95% CI of OR Quart4 | 7.2 | na | 5.7 | 16 | na | 34 | na | 34 | 16 |

Table 5: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

**Chitinase-3-like protein 1**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 251 | 314 | 251 | 570 |

| sCr or UO | 0hr prior to AKI stage | | 24hr prior to AKI stage | |
|---|---|---|---|---|
| | Cohort 1 | Cohort 2 | Cohort 1 | Cohort 2 |
| Average | 543 | 637 | 543 | 1200 |
| Stdev | 1020 | 1120 | 1020 | 1370 |
| p(t-test) | | 0.72 | | 0.016 |
| Min | 18.3 | 14.2 | 18.3 | 12.1 |
| Max | 6000 | 5010 | 6000 | 4740 |
| n (Samp) | 91 | 19 | 91 | 20 |
| n (Patient) | 45 | 19 | 45 | 20 |

| sCr only | 0hr prior to AKI stage | | 24hr prior to AKI stage | |
|---|---|---|---|---|
| | Cohort 1 | Cohort 2 | Cohort 1 | Cohort 2 |
| Median | 277 | 416 | 277 | 3470 |
| Average | 629 | 626 | 629 | 3300 |
| Stdev | 1140 | 715 | 1140 | 1800 |
| p(t-test) | | 1.00 | | 1.2E-4 |
| Min | 12.1 | 38.6 | 12.1 | 1430 |
| Max | 6000 | 1420 | 6000 | 5010 |
| n (Samp) | 123 | 3 | 123 | 3 |
| n (Patient) | 61 | 3 | 61 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | 255 | 314 | 255 | 546 |
| Average | 585 | 685 | 585 | 1190 |
| Stdev | 1060 | 1170 | 1060 | 1410 |
| p(t-test) | | 0.73 | | 0.039 |
| Min | 18.3 | 14.2 | 18.3 | 12.1 |
| Max | 6000 | 5010 | 6000 | 4740 |
| n (Samp) | 84 | 17 | 84 | 19 |
| n (Patient) | 42 | 17 | 42 | 19 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.55 | 0.55 | 0.55 | 0.65 | 0.94 | 0.62 |
| SE | 0.074 | 0.17 | 0.078 | 0.072 | 0.094 | 0.075 |
| p | 0.51 | 0.77 | 0.53 | 0.032 | 2.2E-6 | 0.094 |
| nCohort 1 | 91 | 123 | 84 | 91 | 123 | 84 |
| nCohort 2 | 19 | 3 | 17 | 20 | 3 | 19 |
| Cutoff 1 | 236 | 37.9 | 257 | 303 | 1350 | 192 |
| Sens 1 | 74% | 100% | 71% | 70% | 100% | 74% |
| Spec 1 | 49% | 7% | 51% | 59% | 91% | 37% |
| Cutoff 2 | 48.3 | 37.9 | 76.7 | 139 | 1350 | 104 |
| Sens 2 | 84% | 100% | 82% | 80% | 100% | 84% |
| Spec 2 | 9% | 7% | 10% | 23% | 91% | 13% |

| | 0hr prior to AKI stage | | | 24hr prior to AKI stage | | |
|---|---|---|---|---|---|---|
| | sCr or UO | sCr only | UO only | sCr or UO | sCr only | UO only |
| Cutoff 3 | 37.9 | 37.9 | 38.6 | 37.9 | 1350 | 12.1 |
| Sens 3 | 95% | 100% | 94% | 90% | 100% | 95% |
| Spec 3 | 7% | 7% | 6% | 7% | 91% | 0% |
| Cutoff 4 | 418 | 465 | 443 | 418 | 465 | 443 |
| Sens 4 | 32% | 33% | 35% | 60% | 100% | 58% |
| Spec 4 | 70% | 71% | 70% | 70% | 71% | 70% |
| Cutoff 5 | 513 | 651 | 611 | 513 | 651 | 611 |
| Sens 5 | 32% | 33% | 29% | 60% | 100% | 42% |
| Spec 5 | 80% | 80% | 81% | 80% | 80% | 81% |
| Cutoff 6 | 873 | 1290 | 1290 | 873 | 1290 | 1290 |
| Sens 6 | 11% | 33% | 12% | 45% | 100% | 37% |
| Spec 6 | 90% | 90% | 90% | 90% | 90% | 90% |
| OR Quart 2 | 0.16 | 0 | 0.72 | 0.16 | >0 | 0.16 |
| p Value | 0.11 | na | 0.68 | 0.11 | <na | 0.11 |
| 95% CI of | 0.018 | na | 0.14 | 0.018 | >na | 0.017 |
| OR Quart2 | 1.5 | na | 3.6 | 1.5 | na | 1.5 |
| OR Quart 3 | 1.5 | 1.0 | 1.0 | 0.73 | >0 | 0.73 |
| p Value | 0.51 | 1.0 | 1.0 | 0.67 | <na | 0.67 |
| 95% CI of | 0.42 | 0.060 | 0.22 | 0.17 | >na | 0.17 |
| OR Quart3 | 5.6 | 17 | 4.5 | 3.1 | na | 3.1 |
| OR Quart 4 | 1.2 | 0.97 | 1.6 | 2.4 | >3.2 | 2.1 |
| p Value | 0.79 | 0.98 | 0.53 | 0.16 | <0.32 | 0.25 |
| 95% CI of | 0.32 | 0.058 | 0.39 | 0.71 | >0.32 | 0.59 |
| OR Quart4 | 4.5 | 16 | 6.4 | 8.5 | na | 7.5 |

Table 6: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

**Chitinase-3-like protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 310 | 1180 | 310 | 1180 | 310 | 594 |
| Average | 681 | 1830 | 681 | 1820 | 681 | 2210 |
| Stdev | 1180 | 2020 | 1180 | 2030 | 1180 | 3290 |
| p(t-test) | | 0.055 | | 0.057 | | 0.097 |
| Min | 28.4 | 45.5 | 28.4 | 45.5 | 28.4 | 45.5 |
| Max | 6000 | 6000 | 6000 | 6000 | 6000 | 6000 |
| n (Samp) | 25 | 8 | 25 | 8 | 25 | 3 |
| n (Patient) | 25 | 8 | 25 | 8 | 25 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 338 | 1180 | 338 | 1180 | 338 | 594 |
| Average | 667 | 1830 | 667 | 1820 | 667 | 2210 |
| Stdev | 1160 | 2020 | 1160 | 2030 | 1160 | 3290 |
| p(t-test) | | 0.048 | | 0.049 | | 0.088 |
| Min | 37.9 | 45.5 | 37.9 | 45.5 | 37.9 | 45.5 |
| Max | 6000 | 6000 | 6000 | 6000 | 6000 | 6000 |
| n (Samp) | 26 | 8 | 26 | 8 | 26 | 3 |
| n (Patient) | 26 | 8 | 26 | 8 | 26 | 3 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.74 | nd | 0.74 | 0.73 | nd | 0.73 | 0.59 | nd | 0.60 |
| SE | 0.11 | nd | 0.11 | 0.11 | nd | 0.11 | 0.18 | nd | 0.18 |
| p | 0.032 | nd | 0.031 | 0.037 | nd | 0.035 | 0.61 | nd | 0.60 |
| nCohort 1 | 25 | nd | 26 | 25 | nd | 26 | 25 | nd | 26 |
| nCohort 2 | 8 | nd | 8 | 8 | nd | 8 | 3 | nd | 3 |
| Cutoff 1 | 513 | nd | 513 | 513 | nd | 513 | 37.9 | nd | 38.6 |
| Sens 1 | 75% | nd | 75% | 75% | nd | 75% | 100% | nd | 100% |
| Spec 1 | 72% | nd | 73% | 72% | nd | 73% | 8% | nd | 8% |
| Cutoff 2 | 310 | nd | 310 | 282 | nd | 252 | 37.9 | nd | 38.6 |
| Sens 2 | 88% | nd | 88% | 88% | nd | 88% | 100% | nd | 100% |
| Spec 2 | 52% | nd | 50% | 48% | nd | 46% | 8% | nd | 8% |
| Cutoff 3 | 37.9 | nd | 38.6 | 37.9 | nd | 38.6 | 37.9 | nd | 38.6 |
| Sens 3 | 100% | nd | 100% | 100% | nd | 100% | 100% | nd | 100% |
| Spec 3 | 8% | nd | 8% | 8% | nd | 8% | 8% | nd | 8% |
| Cutoff 4 | 513 | nd | 513 | 513 | nd | 513 | 513 | nd | 513 |
| Sens 4 | 75% | nd | 75% | 75% | nd | 75% | 67% | nd | 67% |
| Spec 4 | 72% | nd | 73% | 72% | nd | 73% | 72% | nd | 73% |
| Cutoff 5 | 873 | nd | 783 | 873 | nd | 783 | 873 | nd | 783 |
| Sens 5 | 50% | nd | 50% | 50% | nd | 50% | 33% | nd | 33% |
| Spec 5 | 80% | nd | 81% | 80% | nd | 81% | 80% | nd | 81% |
| Cutoff 6 | 1350 | nd | 1350 | 1350 | nd | 1350 | 1350 | nd | 1350 |
| Sens 6 | 50% | nd | 50% | 50% | nd | 50% | 33% | nd | 33% |
| Spec 6 | 92% | nd | 92% | 92% | nd | 92% | 92% | nd | 92% |
| OR Quart 2 | 1.0 | nd | 0.88 | 1.0 | nd | 0.88 | 0 | nd | 0 |
| | 1.0 | nd | 0.93 | 1.0 | nd | 0.93 | na | nd | na |
| p Value | 0.052 | nd | 0.046 | 0.052 | nd | 0.046 | na | nd | na |
| 95% CI of OR Quart2 | 19 | nd | 17 | 19 | nd | 17 | na | nd | na |
| OR Quart 3 | 2.3 | nd | 2.3 | 2.3 | nd | 2.3 | 1.0 | nd | 1.0 |
| | 0.53 | nd | 0.53 | 0.53 | nd | 0.53 | 1.0 | nd | 1.0 |
| p Value | 0.17 | nd | 0.17 | 0.17 | nd | 0.17 | 0.050 | nd | 0.050 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart3 | 33 | nd | 33 | 33 | nd | 33 | 20 | nd | 20 |
| OR Quart 4 | 5.6 | nd | 5.6 | 5.6 | nd | 5.6 | 1.0 | nd | 0.86 |
| | 0.17 | nd | 0.17 | 0.17 | nd | 0.17 | 1.0 | nd | 0.92 |
| p Value | 0.47 | nd | 0.47 | 0.47 | nd | 0.47 | 0.050 | nd | 0.044 |
| 95% CI of OR Quart4 | 66 | nd | 66 | 66 | nd | 66 | 20 | nd | 17 |

Table 7: Comparison of marker levels in urine samples collected from Cohort 1 (patients that did not progress beyond RIFLE stage 0, R, or I) and in urine samples collected from Cohort 2 (subjects who progress to RIFLE stage F) at 0, 24 hours, and 48 hours prior to the subject reaching RIFLE stage I.

**Chitinase-3-like protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.30 | 29.2 | 1.30 | 25.2 | nd | nd |
| Average | 15.3 | 47.2 | 15.3 | 45.4 | nd | nd |
| Stdev | 40.9 | 49.7 | 40.9 | 47.1 | nd | nd |
| p(t-test) | | 0.067 | | 0.048 | nd | nd |
| Min | 6.15E-5 | 0.760 | 6.15E-5 | 0.966 | nd | nd |
| Max | 237 | 119 | 237 | 117 | nd | nd |
| n (Samp) | 116 | 6 | 116 | 8 | nd | nd |
| n (Patient) | 56 | 6 | 56 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.24 | 29.2 | 1.24 | 25.2 | nd | nd |
| Average | 16.5 | 47.2 | 16.5 | 45.4 | nd | nd |
| Stdev | 42.6 | 49.7 | 42.6 | 47.1 | nd | nd |
| p(t-test) | | 0.091 | | 0.069 | nd | nd |
| Min | 6.15E-5 | 0.760 | 6.15E-5 | 0.966 | nd | nd |
| Max | 237 | 119 | 237 | 117 | nd | nd |
| n (Samp) | 106 | 6 | 106 | 8 | nd | nd |
| n (Patient) | 51 | 6 | 51 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | nd | 0.79 | 0.80 | nd | 0.79 | nd | nd | nd |
| SE | 0.11 | nd | 0.11 | 0.095 | nd | 0.097 | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.0075 | nd | 0.0097 | 0.0015 | nd | 0.0023 | nd | nd | nd |
| nCohort 1 | 116 | nd | 106 | 116 | nd | 106 | nd | nd | nd |
| nCohort 2 | 6 | nd | 6 | 8 | nd | 8 | nd | nd | nd |
| Cutoff 1 | 7.33 | nd | 7.33 | 9.04 | nd | 9.04 | nd | nd | nd |
| Sens 1 | 83% | nd | 83% | 75% | nd | 75% | nd | nd | nd |
| Spec 1 | 79% | nd | 77% | 81% | nd | 79% | nd | nd | nd |
| Cutoff 2 | 7.33 | nd | 7.33 | 1.88 | nd | 1.84 | nd | nd | nd |
| Sens 2 | 83% | nd | 83% | 88% | nd | 88% | nd | nd | nd |
| Spec 2 | 79% | nd | 77% | 60% | nd | 58% | nd | nd | nd |
| Cutoff 3 | 0.733 | nd | 0.733 | 0.947 | nd | 0.947 | nd | nd | nd |
| Sens 3 | 100% | nd | 100% | 100% | nd | 100% | nd | nd | nd |
| Spec 3 | 34% | nd | 35% | 40% | nd | 41% | nd | nd | nd |
| Cutoff 4 | 3.92 | nd | 5.32 | 3.92 | nd | 5.32 | nd | nd | nd |
| Sens 4 | 83% | nd | 83% | 75% | nd | 75% | nd | nd | nd |
| Spec 4 | 71% | nd | 71% | 71% | nd | 71% | nd | nd | nd |
| Cutoff 5 | 8.95 | nd | 9.23 | 8.95 | nd | 9.23 | nd | nd | nd |
| Sens 5 | 67% | nd | 67% | 75% | nd | 62% | nd | nd | nd |
| Spec 5 | 80% | nd | 80% | 80% | nd | 80% | nd | nd | nd |
| Cutoff 6 | 44.3 | nd | 51.0 | 44.3 | nd | 51.0 | nd | nd | nd |
| Sens 6 | 33% | nd | 33% | 38% | nd | 38% | nd | nd | nd |
| Spec 6 | 91% | nd | 91% | 91% | nd | 91% | nd | nd | nd |
| OR Quart 2 | >1.0 | nd | >1.0 | >1.0 | nd | >1.0 | nd | nd | nd |
| | <1.0 | nd | <0.98 | <0.98 | nd | <1.0 | nd | nd | nd |
| p Value | >0.060 | nd | >0.062 | >0.062 | nd | >0.060 | nd | nd | nd |
| 95% CI of OR Quart2 | na | nd | na | na | nd | na | nd | nd | nd |
| OR Quart 3 | >0 | nd | >1.0 | >1.0 | nd | >1.0 | nd | nd | nd |
| | <na | nd | <0.98 | <0.98 | nd | <0.98 | nd | nd | nd |
| p Value | >na | nd | >0.062 | >0.062 | nd | >0.062 | nd | nd | nd |
| 95% CI of OR Quart3 | na | nd | na | na | nd | na | nd | nd | nd |
| OR Quart 4 | >5.8 | nd | >4.7 | >7.4 | nd | >7.3 | nd | nd | nd |
| | <0.12 | nd | <0.18 | <0.071 | nd | <0.075 | nd | nd | nd |
| p Value | >0.63 | nd | >0.49 | >0.84 | nd | >0.82 | nd | nd | nd |
| 95% CI of OR Quart4 | na | nd | na | na | nd | na | nd | nd | nd |

Table 8: Comparison of marker levels in EDTA samples collected from Cohort 1 (patients that did not progress beyond RIFLE stage 0, R, or I) and in EDTA samples

collected from Cohort 2 (subjects who progress to RIFLE stage F) at 0, 24 hours, and 48 hours prior to the subject reaching RIFLE stage I.

**Chitinase-3-like protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 255 | 455 | 255 | 1180 | nd | nd |
| Average | 600 | 576 | 600 | 1550 | nd | nd |
| Stdev | 1090 | 481 | 1090 | 1440 | nd | nd |
| p(t-test) | | 0.96 | | 0.022 | nd | nd |
| Min | 12.1 | 43.0 | 12.1 | 45.5 | nd | nd |
| Max | 6000 | 1420 | 6000 | 3800 | nd | nd |
| n (Samp) | 116 | 6 | 116 | 8 | nd | nd |
| n (Patient) | 58 | 6 | 58 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 275 | 455 | 275 | 1180 | nd | nd |
| Average | 637 | 576 | 637 | 1550 | nd | nd |
| Stdev | 1130 | 481 | 1130 | 1440 | nd | nd |
| p(t-test) | | 0.90 | | 0.034 | nd | nd |
| Min | 12.1 | 43.0 | 12.1 | 45.5 | nd | nd |
| Max | 6000 | 1420 | 6000 | 3800 | nd | nd |
| n (Samp) | 106 | 6 | 106 | 8 | nd | nd |
| n (Patient) | 53 | 6 | 53 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | nd | 0.62 | 0.75 | nd | 0.74 | nd | nd | nd |
| SE | 0.13 | nd | 0.13 | 0.10 | nd | 0.10 | nd | nd | nd |
| p | 0.28 | nd | 0.33 | 0.014 | nd | 0.020 | nd | nd | nd |
| nCohort 1 | 116 | nd | 106 | 116 | nd | 106 | nd | nd | nd |
| nCohort 2 | 6 | nd | 6 | 8 | nd | 8 | nd | nd | nd |
| Cutoff 1 | 310 | nd | 310 | 521 | nd | 521 | nd | nd | nd |
| Sens 1 | 83% | nd | 83% | 75% | nd | 75% | nd | nd | nd |
| Spec 1 | 59% | nd | 56% | 78% | nd | 76% | nd | nd | nd |
| Cutoff 2 | 310 | nd | 310 | 303 | nd | 303 | nd | nd | nd |
| Sens 2 | 83% | nd | 83% | 88% | nd | 88% | nd | nd | nd |
| Spec 2 | 59% | nd | 56% | 58% | nd | 55% | nd | nd | nd |
| Cutoff 3 | 38.6 | nd | 38.6 | 38.6 | nd | 38.6 | nd | nd | nd |
| Sens 3 | 100% | nd | 100% | 100% | nd | 100% | nd | nd | nd |
| Spec 3 | 9% | nd | 8% | 9% | nd | 8% | nd | nd | nd |
| Cutoff 4 | 443 | nd | 465 | 443 | nd | 465 | nd | nd | nd |
| Sens 4 | 50% | nd | 50% | 75% | nd | 75% | nd | nd | nd |
| Spec 4 | 71% | nd | 71% | 71% | nd | 71% | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 5 | 632 | nd | 651 | 632 | nd | 651 | nd | nd | nd |
| Sens 5 | 33% | nd | 33% | 50% | nd | 50% | nd | nd | nd |
| Spec 5 | 80% | nd | 80% | 80% | nd | 80% | nd | nd | nd |
| Cutoff 6 | 1290 | nd | 1350 | 1290 | nd | 1350 | nd | nd | nd |
| Sens 6 | 17% | nd | 17% | 50% | nd | 50% | nd | nd | nd |
| Spec 6 | 91% | nd | 91% | 91% | nd | 91% | nd | nd | nd |
| OR Quart 2 | 0 | nd | 0 | 0 | nd | 0 | nd | nd | nd |
|  | na | nd | na | na | nd | na | nd | nd | nd |
| p Value | na | nd | na | na | nd | na | nd | nd | nd |
| 95% CI of OR Quart2 | na | nd | na | na | nd | na | nd | nd | nd |
| OR Quart 3 | 2.1 | nd | 2.1 | 2.1 | nd | 2.1 | nd | nd | nd |
|  | 0.56 | nd | 0.56 | 0.56 | nd | 0.56 | nd | nd | nd |
| p Value | 0.18 | nd | 0.18 | 0.18 | nd | 0.18 | nd | nd | nd |
| 95% CI of OR Quart3 | 24 | nd | 24 | 24 | nd | 24 | nd | nd | nd |
| OR Quart 4 | 3.1 | nd | 3.2 | 5.8 | nd | 5.6 | nd | nd | nd |
|  | 0.34 | nd | 0.32 | 0.12 | nd | 0.13 | nd | nd | nd |
| p Value | 0.30 | nd | 0.32 | 0.63 | nd | 0.61 | nd | nd | nd |
| 95% CI of OR Quart4 | 32 | nd | 33 | 53 | nd | 52 | nd | nd | nd |

Table 9: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.

| Chitinase-3-like protein 1 | | | | | | |
|---|---|---|---|---|---|---|
|  | sCr or UO | | sCr only | | UO only | |
|  | Cohort 1 | Cohort 2 | Cohort 1 | Cohort 2 | Cohort 1 | Cohort 2 |
| Median | 1.20 | 7.33 | 1.35 | 52.1 | 0.991 | 7.95 |
| Average | 10.2 | 57.7 | 20.4 | 52.1 | 11.2 | 61.9 |
| Stdev | 25.6 | 85.1 | 50.4 | 63.3 | 26.8 | 87.5 |
| p(t-test) |  | 0.0024 |  | 0.39 |  | 0.0028 |
| Min | 6.15E-5 | 0.341 | 6.15E-5 | 7.33 | 6.15E-5 | 0.341 |
| Max | 138 | 248 | 248 | 96.9 | 138 | 248 |
| n (Samp) | 41 | 13 | 52 | 2 | 37 | 12 |
| n (Patient) | 41 | 13 | 52 | 2 | 37 | 12 |

(continued)

| | At Enrollment | | |
| --- | --- | --- | --- |
| | sCr or UO | sCr only | UO only |
| AUC | 0.72 | 0.85 | 0.71 |
| SE | 0.088 | 0.18 | 0.092 |
| p | 0.013 | 0.048 | 0.022 |
| nCohort 1 | 41 | 52 | 37 |
| nCohort 2 | 13 | 2 | 12 |
| Cutoff 1 | 0.947 | 7.04 | 0.947 |
| Sens 1 | 77% | 100% | 75% |
| Spec 1 | 44% | 77% | 49% |
| Cutoff 2 | 0.875 | 7.04 | 0.875 |
| Sens 2 | 85% | 100% | 83% |
| Spec 2 | 41% | 77% | 46% |
| Cutoff 3 | 0.637 | 7.04 | 0.637 |
| Sens 3 | 92% | 100% | 92% |
| Spec 3 | 34% | 77% | 38% |
| Cutoff 4 | 3.76 | 5.32 | 3.92 |
| Sens 4 | 62% | 100% | 58% |
| Spec 4 | 71% | 71% | 70% |
| Cutoff 5 | 5.79 | 17.3 | 7.33 |
| Sens 5 | 62% | 50% | 50% |
| Spec 5 | 80% | 81% | 81% |
| Cutoff 6 | 18.7 | 62.5 | 33.1 |
| Sens 6 | 38% | 50% | 42% |
| Spec 6 | 90% | 90% | 92% |
| OR Quart 2 | 3.3 | >0 | 3.7 |
| p Value | 0.33 | <na | 0.29 |
| 95% CI of | 0.29 | >na | 0.32 |
| OR Quart2 | 36 | na | 42 |
| OR Quart 3 | 2.2 | >0 | 2.2 |
| p Value | 0.55 | <na | 0.54 |
| 95% CI of | 0.17 | >na | 0.17 |
| OR Quart3 | 28 | na | 28 |
| OR Quart 4 | 12 | >2.2 | 9.4 |
| p Value | 0.034 | <0.55 | 0.058 |
| 95% CI of | 1.2 | >0.17 | 0.93 |
| OR Quart4 | 120 | na | 96 |

Table 10: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.

| Chitinase-3-like protein 1 | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 255 | 1430 | 299 | 2450 | 275 | 1530 |
| Average | 633 | 1860 | 874 | 2450 | 720 | 1890 |
| Stdev | 1290 | 1830 | 1500 | 1440 | 1350 | 1910 |
| p(t-test) | | 0.010 | | 0.15 | | 0.024 |
| Min | 18.3 | 12.1 | 12.1 | 1430 | 18.3 | 12.1 |
| Max | 6000 | 6000 | 6000 | 3470 | 6000 | 6000 |
| n (Samp) | 40 | 13 | 51 | 2 | 36 | 12 |
| n (Patient) | 40 | 13 | 51 | 2 | 36 | 12 |
| | At Enrollment | | | | | |
| | sCr or UO | sCr only | UO only | | | |
| AUC | 0.76 | 0.89 | 0.72 | | | |
| SE | 0.084 | 0.15 | 0.091 | | | |
| p | 0.0020 | 0.010 | 0.014 | | | |
| nCohort 1 | 40 | 51 | 36 | | | |
| nCohort 2 | 13 | 2 | 12 | | | |
| Cutoff 1 | 513 | 1350 | 513 | | | |
| Sens 1 | 77% | 100% | 75% | | | |
| Spec 1 | 78% | 86% | 72% | | | |
| Cutoff 2 | 299 | 1350 | 293 | | | |
| Sens 2 | 85% | 100% | 83% | | | |
| Spec 2 | 60% | 86% | 53% | | | |
| Cutoff 3 | 12.1 | 1350 | 12.1 | | | |
| Sens 3 | 92% | 100% | 92% | | | |
| Spec 3 | 0% | 86% | 0% | | | |
| Cutoff 4 | 466 | 589 | 513 | | | |
| Sens 4 | 77% | 100% | 75% | | | |
| Spec 4 | 70% | 71% | 72% | | | |
| Cutoff 5 | 589 | 969 | 767 | | | |
| Sens 5 | 69% | 100% | 67% | | | |
| Spec 5 | 80% | 80% | 81% | | | |
| Cutoff 6 | 892 | 1770 | 1350 | | | |
| Sens 6 | 69% | 50% | 50% | | | |
| Spec 6 | 90% | 90% | 92% | | | |
| OR Quart 2 | 0 | >0 | 0.45 | | | |

(continued)

| | At Enrollment | | |
|---|---|---|---|
| | sCr or UO | sCr only | UO only |
| p Value | na | <na | 0.54 |
| 95% CI of | na | >na | 0.036 |
| OR Quart2 | na | na | 5.8 |
| OR Quart 3 | 1.0 | >0 | 1.0 |
| p Value | 1.0 | <na | 1.0 |
| 95% CI of | 0.12 | >na | 0.12 |
| OR Quart3 | 8.4 | na | 8.6 |
| OR Quart 4 | 9.9 | >2.2 | 7.0 |
| p Value | 0.016 | <0.55 | 0.045 |
| 95% CI of | 1.5 | >0.17 | 1.0 |
| OR Quart4 | 64 | na | 47 |

The examples provided herein are representative of preferred embodiments and are exemplary.
Thus, for example, in each instance herein any of the terms "comprising", "consisting essentially of" and "consisting of'
may be replaced with either of the other two terms.

**Claims**

1. A method for determining a subject's risk for progression to ARF, comprising:

performing an assay that generates a detectable signal indicative of the amount of a physiologically relevant concentration of Chitinase-3-like protein 1 on a body fluid sample obtained from the subject to provide an assay result; and
correlating the assay result to the renal status of the subject, wherein said correlation step comprises correlating the assay result to a likelihood of one or more future changes in renal status, said future changes in renal status comprising future acute renal failure (ARF),
and wherein the correlating comprises comparing an assay result in the form of the Chitinase-3-like protein 1 concentration to a predetermined threshold selected to be indicative of the likelihood of future ARF,
wherein an increased likelihood of progression to ARF is assigned to the subject, when the measured concentration is above the threshold relative to a likelihood assigned when the measured concentration is below the threshold,
and wherein the likelihood of future ARF is more or less likely to occur within 48 hours of the time at which the body fluid sample is obtained from the subject.

2. A method according to claim 1, wherein a plurality of assay results are combined using a function that converts the plurality of assay results into a single composite result.

3. A method according to claim 1 or 2, wherein the likelihood of one or more future changes in renal status is that an event of interest is more or less likely to occur within a period selected from the group consisting of 36 hours, 24 hours, 18 hours, and 12 hours.

4. A method according to one of claims 1-3, wherein the subject is selected for evaluation of renal status based on the pre-existence in the subject of one or more known risk factors for prerenal, intrinsic renal, or postrenal ARF

5. A method according to one of claims 1-3, wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

6. A method according to one of claims 1-4, wherein said correlating step comprises assessing whether or not renal function is improving or worsening in a subject who has suffered from an injury to renal function or reduced renal function based on the assay result(s), or
wherein said method is a method of assigning a risk of the future occurrence or nonoccurrence of an injury to renal function in said subject, or
wherein said method is a method of assigning a risk of the future occurrence or nonoccurrence of reduced renal function in said subject, or
wherein said method is a method of assigning a risk of the future occurrence or nonoccurrence of a need for dialysis in said subject, or
wherein said method is a method of assigning a risk of the future occurrence or nonoccurrence of acute renal failure in said subject, or
wherein said method is a method of assigning a risk of the future occurrence or nonoccurrence of a need for renal replacement therapy in said subject, or
wherein said method is a method of assigning a risk of the future occurrence or nonoccurrence of a need for renal transplantation in said subject, or
wherein said one or more future changes in renal status comprise one or more of a future injury to renal function, future reduced renal function, future improvement in renal function, and future acute renal failure (ARF) within 48 hours of the time at which the body fluid sample is obtained,
wherein said one or more future changes in renal status preferably comprise one or more of a future injury to renal function, future reduced renal function, future improvement in renal function, and future acute renal failure (ARF) within 24 hours of the time at which the body fluid sample is obtained,
wherein said one or more future changes in renal status preferably comprise one or more of a future injury to renal function, future reduced renal function, future improvement in renal function, and future acute renal failure (ARF) within 18 hours of the time at which the body fluid sample is obtained.

7. A method according to one of claims 1-6, wherein the subject is in RIFLE stage 0 or R.

8. A method according to claim 7, wherein the subject is in RIFLE stage 0, and said correlating step comprises assigning a likelihood that the subject will reach RIFLE stage R, I or F within 48 hours, 36 hours, 24 hours, 18 hours, or 12 hours, or
wherein the subject is in RIFLE stage 0, and said correlating step comprises assigning a likelihood that the subject will reach RIFLE stage I or F within 48 hours, 36 hours, 24 hours, 18 hours, or 12 hours, or
wherein the subject is in RIFLE stage 0, and said correlating step comprises assigning a likelihood that the subject will reach RIFLE stage F within 48 hours, 36 hours, 24 hours, 18 hours, or 12 hours, or
wherein the subject is in RIFLE stage 0 or R, and said correlating step comprises assigning a likelihood that the subject will reach RIFLE stage I or F within 48 hours, 36 hours, 24 hours, 18 hours, or 12 hours, or
wherein the subject is in RIFLE stage 0 or R, and said correlating step comprises assigning a likelihood that the subject will reach RIFLE stage F within 48 hours, 36 hours, 24 hours, 18 hours, or 12 hours, or
wherein the subject is in RIFLE stage R, and said correlating step comprises assigning a likelihood that the subject will reach RIFLE stage I or F within 48 hours, 36 hours, 24 hours, 18 hours, or 12 hours, or
wherein the subject is in RIFLE stage R, and said correlating step comprises assigning a likelihood that the subject will reach RIFLE stage F within 48 hours, 36 hours, 24 hours, 18 hours, or 12 hours.

9. A method according to one of claims 1-6, wherein the subject is in RIFLE stage 0, R, or I, and said correlating step comprises assigning a likelihood that the subject will reach RIFLE stage F within 48 hours, 36 hours, 24 hours, 18 hours, or 12 hours, wherein the subject preferably is in RIFLE stage I, and said correlating step comprises assigning a likelihood that the subject will reach RIFLE stage F within 48 hours, 36 hours, 24 hours, 18 hours, or 12 hours, or
wherein the subject is not in acute renal failure, or
wherein the subject has not experienced a 1.5-fold or greater increase in serum creatinine over a baseline value determined prior to the time at which the body fluid sample is obtained, or
wherein the subject has a urine output of at least 0.5 ml/kg/hr over the 6 hours preceding the time at which the body fluid sample is obtained, or
wherein the subject has not experienced an increase of 0.3 mg/dL or greater in serum creatinine over a baseline value determined prior to the time at which the body fluid sample is obtained, or
wherein the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained.

10. A method according to one of claims 1-6,
wherein said correlating step comprises assigning one or more of: a likelihood that within 48 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine,
wherein said correlating step preferably comprises assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine,
wherein said correlating step preferably comprises assigning one or more of: a likelihood that within 18 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

11. A method according to one of claims 1-6, wherein the subject has not experienced a 2-fold or greater increase in serum creatinine over a baseline value determined prior to the time at which the body fluid sample is obtained, or
wherein the subject has a urine output of at least 0.5 ml/kg/hr over the 12 hours preceding the time at which the body fluid sample is obtained, or
wherein said correlating step preferably comprises assigning a likelihood that within 48 hours the subject will experience a 2-fold or greater increase in serum creatinine,
wherein said correlating step preferably comprises assigning a likelihood that within 24 hours the subject will experience a 2-fold or greater increase in serum creatinine, or
wherein said correlating step preferably comprises assigning a likelihood that within 18 hours the subject will experience a 2-fold or greater increase in serum creatinine.

12. A method according to one of claims 1-6, wherein said correlating step comprises assigning one or more of: a likelihood that within 48 hours the subject will (i) experience a 3-fold or greater increase in serum creatinine, or (ii) have a urine output of less than 0.3 ml/kg/hr over a 24 hour period or anuria over a 12 hour period,
wherein said correlating step preferably comprises assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period,
wherein said correlating step preferably comprises assigning one or more of: a likelihood that within 18 hours the subject will (i) experience a 3-fold or greater increase in serum creatinine, or (ii) have a urine output of less than 0.3 ml/kg/hr over a 24 hour period or anuria over a 12 hour period.

13. A method for evaluating renal status in a subject, comprising:

performing an assay that generates a detectable signal indicative of the amount of a physiologically relevant concentration of Chitinase-3-like protein 1 on a body fluid sample obtained from the subject to provide an assay result; and
correlating the assay result to the renal status of the subject, wherein said correlation step comprises correlating the assay result to a likelihood of one or more future changes in renal status, wherein the future change is future improvement in renal function, and wherein the correlating comprises comparing an assay result in the form of the Chitinase-3-like protein 1 concentration to a predetermined threshold selected to be indicative of the likelihood of future improvement of renal function,
wherein an increased likelihood of a future improvement in renal function is assigned to the subject when the measured concentration is below the threshold, relative to a likelihood assigned when the measured concentration is above the threshold.

14. A method according to one of claims 1-13, wherein the body fluid sample is a urine sample.

15. A method according to claim 13, wherein said correlating step comprises assessing whether or not renal function is improving in a subject who has suffered from an injury to renal function, reduced renal function, or ARF based on the assay result.

**16.** A method according to claim 13, wherein a plurality of assay results are combined using a function that converts the plurality of assay results into a single composite result.

**17.** Use of a measured concentration of Chitinase-3-like protein 1 on a body fluid sample obtained from a subject for assigning a likelihood of one or more future changes in renal status to the subject, selected from the group consisting of a future improvement in renal function and future acute renal failure (ARF), the use comprising
comparing a measured concentration of Chitinase-3-like protein 1 to a threshold value, wherein an increased likelihood of a future improvement in renal function is assigned to the subject when the measured concentration is below the threshold, relative to a likelihood assigned when the measured concentration is above the threshold, or
wherein an increased likelihood of progression to ARF is assigned to the subject, when the measured concentration is above the threshold relative to a likelihood assigned when the measured concentration is below the threshold, and wherein the likelihood of future ARF is more or less likely to occur within 48 hours of the time at which the body fluid sample is obtained from the subject.

**Patentansprüche**

**1.** Ein Verfahren zur Bestimmung des Risikos eines Subjektes für eine Progression zu ARF, das Folgendes umfasst:

Durchführen einer Untersuchung, die ein nachweisbares Signal erzeugt, das die Menge einer physiologisch relevanten Konzentration von Chitinase-3-like-Protein 1 in einer von dem Subjekt erhaltenen Körperflüssigkeitsprobe anzeigt, um ein Untersuchungsergebnis bereitzustellen; und
Korrelieren des Untersuchungsergebnisses mit dem Nierenstatus des Subjektes, wobei der Korrelierungsschritt das Korrelieren des Untersuchungsergebnisses mit einer Wahrscheinlichkeit einer oder mehrerer zukünftiger Veränderungen des Nierenstatus umfasst, wobei die zukünftigen Veränderungen des Nierenstatus zukünftiges akutes Nierenversagen (ARF) umfassen,
und wobei das Korrelieren das Vergleichen eines Untersuchungsergebnisses in Form der Chitinase-3-like-Protein-1-Konzentration mit einem vorbestimmten Schwellenwert umfasst, der so ausgewählt ist, dass er die Wahrscheinlichkeit eines zukünftigen ARF anzeigt,
wobei dem Subjekt eine erhöhte Wahrscheinlichkeit einer Progression zu ARF zugewiesen wird, wenn die gemessene Konzentration über dem Schwellenwert liegt, relativ zu einer Wahrscheinlichkeit, die zugewiesen wird, wenn die gemessene Konzentration unter dem Schwellenwert liegt,
und wobei die Wahrscheinlichkeit eines zukünftigen ARF mehr oder weniger wahrscheinlich innerhalb von 48 Stunden nach dem Zeitpunkt auftritt, an dem die Körperflüssigkeitsprobe von dem Subjekt erhalten wurde.

**2.** Verfahren nach Anspruch 1, wobei eine Vielzahl von Untersuchungsergebnissen unter Verwendung einer Funktion kombiniert wird, die die Vielzahl von Untersuchungsergebnissen in ein einziges zusammengesetztes Ergebnis umwandelt.

**3.** Verfahren nach Anspruch 1 oder 2, wobei die Wahrscheinlichkeit einer oder mehrerer zukünftiger Veränderungen des Nierenstatus darin besteht, dass ein Ereignis von Interesse mehr oder weniger wahrscheinlich innerhalb eines Zeitraums auftritt, der aus der Gruppe ausgewählt ist, die aus 36 Stunden, 24 Stunden, 18 Stunden und 12 Stunden besteht.

**4.** Verfahren nach einem der Ansprüche 1-3, wobei das Subjekt, basierend auf dem vorherigen Bestehen eines oder mehrerer bekannter Risikofaktoren für prärenales, immanent renales oder postrenales ARF bei dem Subjekt, zur Evaluierung des Nierenstatus ausgewählt wird.

**5.** Verfahren nach einem der Ansprüche 1-3, wobei das Subjekt, basierend auf einer vorhandenen Diagnose eines oder mehreren von kongestiver Herzinsuffizienz, Präeklampsie, Eklampsie, Diabetes mellitus, Hypertonie, koronarer Herzkrankheit, Proteinurie, Niereninsuffizienz, glomerulärer Filtration unterhalb des normalen Bereichs, Zirrhose, Serumkreatinin oberhalb des normalen Bereichs, Sepsis, Schädigung der Nierenfunktion, reduzierter Nierenfunktion oder basierend auf einem laufenden oder vorangegangenen größeren gefäßchirurgischen Eingriff, Koronararterien-Bypassoperation oder einer anderen Herzoperation oder basierend auf einer Exposition gegenüber NSAIDs, Cyclosporinen, Tacrolimus, Aminoglykosiden, Foscarnet, Ethylenglykol, Hämoglobin, Myoglobin, Ifosfamid, Schwermetallen, Methotrexat, röntgendichten Kontrastmitteln oder Streptozotocin, zur Evaluierung des Nierenstatus ausgewählt wird.

**6.** Verfahren nach einem der Ansprüche 1-4, wobei der Korrelierungsschritt, basierend auf dem/den Untersuchungsergebnis(sen), das Beurteilen umfasst, ob sich die Nierenfunktion bei einem Subjekt, das eine Schädigung der Nierenfunktion oder eine reduzierte Nierenfunktion erlitten hat, verbessert oder verschlechtert, oder

wobei das Verfahren ein Verfahren zum Zuweisen eines Risikos für das zukünftige Auftreten oder Nichtauftreten einer Schädigung der Nierenfunktion bei dem Subjekt ist oder

wobei das Verfahren ein Verfahren zum Zuweisen eines Risikos für das zukünftige Auftreten oder Nichtauftreten einer reduzierten Nierenfunktion bei dem Subjekt ist oder

wobei das Verfahren ein Verfahren zum Zuweisen eines Risikos für das zukünftige Auftreten oder Nichtauftreten eines Dialysebedarfs bei dem Subjekt ist oder

wobei das Verfahren ein Verfahren zum Zuweisen eines Risikos für das zukünftige Auftreten oder Nichtauftreten eines akuten Nierenversagens bei dem Subjekt ist oder

wobei das Verfahren ein Verfahren zum Zuweisen eines Risikos für das zukünftige Auftreten oder Nichtauftreten eines Bedarfs einer Nierenersatztherapie bei dem Subjekt ist oder

wobei das Verfahren ein Verfahren zum Zuweisen eines Risikos für das zukünftige Auftreten oder Nichtauftreten eines Bedarfs einer Nierentransplantation bei dem Subjekt ist oder

wobei die eine oder mehreren zukünftigen Veränderungen des Nierenstatus eine oder mehrere von einer zukünftigen Schädigung der Nierenfunktion, zukünftigen reduzierten Nierenfunktion, zukünftigen Verbesserung der Nierenfunktion und zukünftigem akutem Nierenversagen (ARF) innerhalb von 48 Stunden nach dem Zeitpunkt, an dem die Körperflüssigkeitsprobe erhalten wurde, umfassen,

wobei die eine oder mehreren zukünftigen Veränderungen des Nierenstatus bevorzugt eine oder mehrere von einer zukünftigen Schädigung der Nierenfunktion, zukünftigen reduzierten Nierenfunktion, zukünftigen Verbesserung der Nierenfunktion und zukünftigem akutem Nierenversagen (ARF) innerhalb von 24 Stunden nach dem Zeitpunkt, an dem die Körperflüssigkeitsprobe erhalten wurde, umfassen,

wobei die eine oder mehreren zukünftigen Veränderungen des Nierenstatus bevorzugt eine oder mehrere von einer zukünftigen Schädigung der Nierenfunktion, zukünftigen reduzierten Nierenfunktion, zukünftigen Verbesserung der Nierenfunktion und zukünftigem akutem Nierenversagen (ARF) innerhalb von 18 Stunden nach dem Zeitpunkt, an dem die Körperflüssigkeitsprobe erhalten wurde, umfassen.

**7.** Verfahren nach einem der Ansprüche 1-6, wobei das Subjekt im RIFLE-Stadium 0 oder R ist.

**8.** Verfahren nach Anspruch 7, wobei das Subjekt im RIFLE-Stadium 0 ist und der Korrelierungsschritt das Zuweisen einer Wahrscheinlichkeit, dass das Subjekt innerhalb von 48 Stunden, 36 Stunden, 24 Stunden, 18 Stunden oder 12 Stunden das RIFLE-Stadium R, I oder F erreichen wird, umfasst oder

wobei das Subjekt im RIFLE-Stadium 0 ist und der Korrelierungsschritt das Zuweisen einer Wahrscheinlichkeit, dass das Subjekt innerhalb von 48 Stunden, 36 Stunden, 24 Stunden, 18 Stunden oder 12 Stunden das RIFLE-Stadium I oder F erreichen wird, umfasst oder

wobei das Subjekt im RIFLE-Stadium 0 ist und der Korrelierungsschritt das Zuweisen einer Wahrscheinlichkeit, dass das Subjekt innerhalb von 48 Stunden, 36 Stunden, 24 Stunden, 18 Stunden oder 12 Stunden das RIFLE-Stadium F erreichen wird, umfasst oder

wobei das Subjekt im RIFLE-Stadium 0 oder R ist und der Korrelierungsschritt das Zuweisen einer Wahrscheinlichkeit, dass das Subjekt innerhalb von 48 Stunden, 36 Stunden, 24 Stunden, 18 Stunden oder 12 Stunden das RIFLE-Stadium I oder F erreichen wird, umfasst oder

wobei das Subjekt im RIFLE-Stadium 0 oder R ist und der Korrelierungsschritt das Zuweisen einer Wahrscheinlichkeit, dass das Subjekt innerhalb von 48 Stunden, 36 Stunden, 24 Stunden, 18 Stunden oder 12 Stunden das RIFLE-Stadium F erreichen wird, umfasst oder

wobei das Subjekt im RIFLE-Stadium R ist und der Korrelierungsschritt das Zuweisen einer Wahrscheinlichkeit, dass das Subjekt innerhalb von 48 Stunden, 36 Stunden, 24 Stunden, 18 Stunden oder 12 Stunden das RIFLE-Stadium I oder F erreichen wird, umfasst oder

wobei das Subjekt im RIFLE-Stadium R ist und der Korrelierungsschritt das Zuweisen einer Wahrscheinlichkeit, dass das Subjekt innerhalb von 48 Stunden, 36 Stunden, 24 Stunden, 18 Stunden oder 12 Stunden das RIFLE-Stadium F erreichen wird, umfasst.

**9.** Verfahren nach einem der Ansprüche 1-6, wobei das Subjekt im RIFLE-Stadium 0, R oder I ist und der Korrelierungsschritt das Zuweisen einer Wahrscheinlichkeit, dass das Subjekt innerhalb von 48 Stunden, 36 Stunden, 24 Stunden, 18 Stunden oder 12 Stunden das RIFLE-Stadium F erreichen wird, umfasst,

wobei das Subjekt bevorzugt im RIFLE-Stadium I ist und der Korrelierungsschritt das Zuweisen einer Wahrscheinlichkeit, dass das Subjekt innerhalb von 48 Stunden, 36 Stunden, 24 Stunden, 18 Stunden oder 12 Stunden das RIFLE-Stadium F erreichen wird, umfasst oder

wobei das Subjekt kein akutes Nierenversagen hat oder

wobei das Subjekt keine 1,5-fache oder größere Zunahme des Serumkreatinins über einen Basiswert, der vor dem Zeitpunkt, an dem die Körperflüssigkeitsprobe erhalten wurde, bestimmt wurde, erfahren hat oder

wobei das Subjekt eine Urinausscheidung von mindestens 0,5 ml/kg/Std. über die 6 Stunden vor dem Zeitpunkt, an dem die Körperflüssigkeitsprobe erhalten wurde, hat oder

wobei das Subjekt keine Zunahme von 0,3 mg/dl oder mehr des Serumkreatinins über einen Basiswert, der vor dem Zeitpunkt, an dem die Körperflüssigkeitsprobe erhalten wurde, bestimmt wurde, erfahren hat oder

wobei das Subjekt (i) keine 1,5-fache oder größere Zunahme des Serumkreatinins über einen Basiswert, der vor dem Zeitpunkt, an dem die Körperflüssigkeitsprobe erhalten wurde, bestimmt wurde, erfahren hat, (ii) eine Urinausscheidung von mindestens 0,5 ml/kg/Std. über die 6 Stunden vor dem Zeitpunkt, an dem die Körperflüssigkeitsprobe erhalten wurde, hat und (iii) keine Zunahme von 0,3 mg/dl oder mehr des Serumkreatinins über einen Basiswert, der vor dem Zeitpunkt, an dem die Körperflüssigkeitsprobe erhalten wurde, bestimmt wurde, erfahren hat.

10. Verfahren nach einem der Ansprüche 1-6,

wobei der Korrelierungsschritt das Zuweisen von einem oder mehreren von: einer Wahrscheinlichkeit, dass das Subjekt innerhalb von 48 Stunden (i) eine 1,5-fache oder größere Zunahme des Serumkreatinins erfahren wird, (ii) eine Urinausscheidung von weniger als 0,5 ml/kg/Std. über einen Zeitraum von 6 Stunden haben wird oder (iii) eine Zunahme des Serumkreatinins von 0,3 mg/dl oder mehr erfahren wird, umfasst,

wobei der Korrelierungsschritt bevorzugt das Zuweisen von einem oder mehreren von: einer Wahrscheinlichkeit, dass das Subjekt innerhalb von 24 Stunden (i) eine 1,5-fache oder größere Zunahme des Serumkreatinins erfahren wird, (ii) eine Urinausscheidung von weniger als 0,5 ml/kg/Std. über einen Zeitraum von 6 Stunden haben wird oder (iii) eine Zunahme des Serumkreatinins von 0,3 mg/dl oder mehr erfahren wird, umfasst,

wobei der Korrelierungsschritt bevorzugt das Zuweisen von einem oder mehreren von: einer Wahrscheinlichkeit, dass das Subjekt innerhalb von 18 Stunden (i) eine 1,5-fache oder größere Zunahme des Serumkreatinins erfahren wird, (ii) eine Urinausscheidung von weniger als 0,5 ml/kg/Std. über einen Zeitraum von 6 Stunden haben wird oder (iii) eine Zunahme des Serumkreatinins von 0,3 mg/dl oder mehr erfahren wird, umfasst.

11. Verfahren nach einem der Ansprüche 1-6, wobei das Subjekt keine 2-fache oder größere Zunahme des Serumkreatinins über einen Basiswert, der vor dem Zeitpunkt bestimmt wurde, an dem die Körperflüssigkeitsprobe erhalten wurde, erfahren hat oder

wobei das Subjekt eine Urinausscheidung von mindestens 0,5 ml/kg/Std. über die 12 Stunden vor dem Zeitpunkt, an dem die Körperflüssigkeitsprobe erhalten wurde, hat oder

wobei der Korrelierungsschritt bevorzugt das Zuweisen einer Wahrscheinlichkeit, dass das Subjekt innerhalb von 48 Stunden eine 2-fache oder größere Zunahme des Serumkreatinins erfahren hat, umfasst,

wobei der Korrelierungsschritt bevorzugt das Zuweisen einer Wahrscheinlichkeit, dass das Subjekt innerhalb von 24 Stunden eine 2-fache oder größere Zunahme des Serumkreatinins erfahren wird, umfasst oder

wobei der Korrelierungsschritt bevorzugt das Zuweisen einer Wahrscheinlichkeit, dass das Subjekt innerhalb von 18 Stunden eine 2-fache oder größere Zunahme des Serumkreatinins erfahren wird, umfasst.

12. Verfahren nach einem der Ansprüche 1-6, wobei der Korrelierungsschritt das Zuweisen von einem oder mehreren von: einer Wahrscheinlichkeit, dass das Subjekt innerhalb von 48 Stunden (i) eine 3-fache oder größere Zunahme des Serumkreatinins erfahren wird oder (ii) eine Urinausscheidung von weniger als 0,3 ml/kg/Std. über einen Zeitraum von 24 Stunden oder Anurie über einen Zeitraum von 12 Stunden haben wird, umfasst,

wobei der Korrelierungsschritt bevorzugt das Zuweisen von einem oder mehreren von: einer Wahrscheinlichkeit, dass das Subjekt innerhalb von 24 Stunden (i) eine 3-fache oder größere Zunahme des Serumkreatinins erfahren wird oder (ii) eine Urinausscheidung von weniger als 0,3 ml/kg/Std. über einen Zeitraum von 24 Stunden oder Anurie über einen Zeitraum von 12 Stunden haben wird, umfasst,

wobei der Korrelierungsschritt bevorzugt das Zuweisen von einem oder mehreren von: einer Wahrscheinlichkeit, dass das Subjekt innerhalb von 18 Stunden (i) eine 3-fache oder größere Zunahme des Serumkreatinins erfahren wird oder (ii) eine Urinausscheidung von weniger als 0,3 ml/kg/Std. über einen Zeitraum von 24 Stunden oder Anurie über einen Zeitraum von 12 Stunden haben wird.

13. Ein Verfahren zur Evaluierung des Nierenstatus eines Subjektes, umfassend:

Durchführen einer Untersuchung, die ein nachweisbares Signal erzeugt, das die Menge einer physiologisch relevanten Konzentration von Chitinase-3-like-Protein 1 in einer von dem Subjekt erhaltenen Körperflüssigkeitsprobe anzeigt, um ein Untersuchungsergebnis bereitzustellen; und

Korrelieren des Untersuchungsergebnisses mit dem Nierenstatus des Subjektes, wobei der Korrelierungsschritt das Korrelieren des Untersuchungsergebnisses mit einer Wahrscheinlichkeit einer oder mehrerer zukünftiger Veränderungen des Nierenstatus umfasst, wobei die zukünftige Veränderung die zukünftige Verbesserung der Nierenfunktion ist, und

wobei das Korrelieren das Vergleichen eines Untersuchungsergebnisses in Form der Chitinase-3-like-Protein-1-Konzentration mit einem vorbestimmten Schwellenwert umfasst, der so ausgewählt ist, dass er die Wahrscheinlichkeit einer zukünftigen Verbesserung der Nierenfunktion anzeigt,

wobei dem Subjekt eine erhöhte Wahrscheinlichkeit einer zukünftigen Verbesserung der Nierenfunktion zugewiesen wird, wenn die gemessene Konzentration unter dem Schwellenwert liegt, relativ zu einer Wahrscheinlichkeit, die zugewiesen wird, wenn die gemessene Konzentration über dem Schwellenwert liegt.

14. Verfahren nach einem der Ansprüche 1-13, wobei die Körperflüssigkeitsprobe eine Urinprobe ist.

15. Verfahren nach Anspruch 13, wobei der Korrelierungsschritt das Beurteilen umfasst, ob sich, basierend auf dem Untersuchungsergebnis, die Nierenfunktion bei einem Subjekt, das eine Schädigung der Nierenfunktion, eine reduzierte Nierenfunktion oder ARF erlitten hat, verbessert oder nicht.

16. Verfahren nach Anspruch 13, wobei eine Vielzahl von Untersuchungsergebnissen unter Verwendung einer Funktion kombiniert wird, die die Vielzahl von Untersuchungsergebnissen in ein einziges zusammengesetztes Ergebnis umwandelt.

17. Verwendung einer gemessenen Konzentration von Chitinase-3-like-Protein 1 in einer von einem Subjekt erhaltenen Körperflüssigkeitsprobe zum Zuweisen einer Wahrscheinlichkeit einer oder mehrerer zukünftiger Veränderungen des Nierenstatus zu dem Subjekt, die aus der Gruppe ausgewählt sind, die aus einer zukünftigen Verbesserung der Nierenfunktion und zukünftigem akutem Nierenversagen (ARF) besteht, wobei die Verwendung das Vergleichen einer gemessenen Konzentration von Chitinase-3-like-Protein 1 mit einem Schwellenwert umfasst,

wobei dem Subjekt eine erhöhte Wahrscheinlichkeit einer zukünftigen Verbesserung der Nierenfunktion zugewiesen wird, wenn die gemessene Konzentration unter dem Schwellenwert liegt, relativ zu einer Wahrscheinlichkeit, die zugewiesen wird, wenn die gemessene Konzentration über dem Schwellenwert liegt, oder

wobei dem Subjekt eine erhöhte Wahrscheinlichkeit einer Progression zu ARF zugewiesen wird, wenn die gemessene Konzentration über dem Schwellenwert liegt, relativ zu einer Wahrscheinlichkeit, die zugewiesen wird, wenn die gemessene Konzentration unter dem Schwellenwert liegt,

und wobei die Wahrscheinlichkeit eines zukünftigen ARF mehr oder weniger wahrscheinlich innerhalb von 48 Stunden nach dem Zeitpunkt auftritt, an dem die Körperflüssigkeitsprobe von dem Subjekt erhalten wurde.

## Revendications

1. Procédé permettant de déterminer le risque d'évolution d'un sujet jusqu'à une IRA, comprenant :

la réalisation d'une analyse qui produit un signal détectable indiquant le niveau d'une concentration physiologiquement pertinente de protéine 1 similaire à la chitinase-3 sur un échantillon de fluide corporel obtenu sur le sujet pour fournir un résultat d'analyse ; et

la corrélation du résultat d'analyse à l'état rénal du sujet, ladite étape de corrélation comprenant une corrélation du résultat d'analyse à une probabilité d'un ou plusieurs changements futurs de l'état rénal, lesdits changements futurs de l'état rénal comprenant une insuffisance rénale aiguë (IRA) future,

et dans lequel la corrélation comprend une comparaison d'un résultat d'analyse sous la forme de la concentration de protéine 1 similaire à la chitinase-3 à un seuil prédéterminé sélectionné pour indiquer la probabilité d'IRA future,

dans lequel une probabilité accrue d'évolution jusqu'à une IRA est assignée au sujet lorsque la concentration mesurée est supérieure au seuil, par rapport à une probabilité assignée lorsque la concentration mesurée est inférieure au seuil,

et dans lequel la probabilité d'IRA future est plus ou moins élevée dans les 48 heures suivant le moment où l'échantillon de fluide corporel est obtenu sur le sujet.

2. Procédé selon la revendication 1, dans lequel une pluralité de résultats d'analyse sont combinés en utilisant une fonction qui convertit la pluralité de résultats d'analyse en un seul résultat composite.

3. Procédé selon la revendication 1 ou 2, dans lequel la probabilité d'un ou plusieurs changements futurs de l'état rénal est qu'un événement d'intérêt est plus ou moins susceptible de se produire au cours d'une période sélectionnée dans le groupe constitué de 36 heures, 24 heures, 18 heures et 12 heures.

4. Procédé selon l'une des revendications 1 à 3, dans lequel le sujet est sélectionné pour une évaluation de l'état rénal sur la base de la préexistence chez le sujet d'un ou plusieurs facteurs de risque connus pour une IRA pré-rénale, rénale intrinsèque, ou post-rénale.

5. Procédé selon l'une des revendications 1 à 3, dans lequel le sujet est sélectionné pour une évaluation de l'état rénal sur la base d'un diagnostic existant d'une ou plusieurs pathologies parmi une insuffisance cardiaque congestive, une pré-éclampsie, une éclampsie, un diabète sucré, une hypertension, une coronaropathie, une protéinurie, une insuffisance rénale, une filtration glomérulaire inférieure à la plage normale, une cirrhose, une créatinine sérique supérieure à la plage normale, un sepsis, une lésion de la fonction rénale, une fonction rénale réduite, ou sur la base du fait qu'il doit subir ou a subi une intervention chirurgicale vasculaire majeure, un pontage aorto-coronarien, ou une autre intervention cardiaque, ou sur la base d'une exposition à des AINS, à des cyclosporines, au tacrolimus, à des aminoglycosides, au foscarnet, à l'éthylène glycol, à l'hémoglobine, à la myoglobine, à l'ifosfamide, à des métaux lourds, au méthotrexate, à des agents de contraste radio-opaques, ou à la streptozotocine.

6. Procédé selon l'une des revendications 1 à 4, dans lequel ladite étape de corrélation comprend le fait d'évaluer si la fonction rénale s'améliore ou s'aggrave ou non chez un sujet qui a souffert d'une lésion de la fonction rénale, ou d'une fonction rénale réduite sur la base du/des résultat(s) d'analyse, ou
ledit procédé étant un procédé d'assignation d'un risque de l'occurrence ou non-occurrence future d'une lésion de la fonction rénale chez ledit sujet, ou
ledit procédé étant un procédé d'assignation d'un risque de l'occurrence ou non-occurrence future d'une fonction rénale réduite chez ledit sujet, ou
ledit procédé étant un procédé d'assignation d'un risque de l'occurrence ou non-occurrence future d'un besoin de dialyse chez ledit sujet, ou
ledit procédé étant un procédé d'assignation d'un risque de l'occurrence ou non-occurrence future d'une insuffisance rénale aiguë chez ledit sujet, ou
ledit procédé étant un procédé d'assignation d'un risque de l'occurrence ou non-occurrence future d'un besoin de traitement rénal substitutif chez ledit sujet, ou
ledit procédé étant un procédé d'assignation d'un risque de l'occurrence ou non-occurrence future d'un besoin de transplantation rénale chez ledit sujet, ou
dans lequel lesdits un ou plusieurs changements futurs de l'état rénal comprennent une ou plusieurs d'une lésion future de la fonction rénale, d'une fonction rénale réduite future, d'une amélioration future de la fonction rénale, et d'une insuffisance rénale aiguë (IRA) future dans les 48 heures du moment où l'échantillon de fluide corporel est obtenu,
dans lequel lesdits un ou plusieurs changements futurs de l'état rénal comprennent préférablement une ou plusieurs d'une lésion future de la fonction rénale, d'une fonction rénale réduite future, d'une amélioration future de la fonction rénale, et d'une insuffisance rénale aiguë (IRA) future dans les 24 heures du moment où l'échantillon de fluide corporel est obtenu,
dans lequel lesdits un ou plusieurs changements futurs de l'état rénal comprennent préférablement une ou plusieurs d'une lésion future de la fonction rénale, d'une fonction rénale réduite future, d'une amélioration future de la fonction rénale, et d'une insuffisance rénale aiguë (IRA) future dans les 18 heures du moment où l'échantillon de fluide corporel est obtenu.

7. Procédé selon l'une des revendications 1 à 6, dans lequel le sujet est dans la classe 0 ou R de RIFLE.

8. Procédé selon la revendication 7, dans lequel le sujet est dans la classe 0 de RIFLE, et ladite étape de corrélation comprend l'assignation d'une probabilité que le sujet atteindra la classe R, I ou F de RIFLE dans les 48 heures, 36 heures, 24 heures, 18 heures, ou 12 heures, ou
dans lequel le sujet est dans la classe 0 de RIFLE, et ladite étape de corrélation comprend l'assignation d'une probabilité que le sujet atteindra la classe I ou F de RIFLE dans les 48 heures, 36 heures, 24 heures, 18 heures, ou 12 heures, ou
dans lequel le sujet est dans la classe 0 de RIFLE, et ladite étape de corrélation comprend l'assignation d'une probabilité que le sujet atteindra la classe F de RIFLE dans les 48 heures, 36 heures, 24 heures, 18 heures, ou 12 heures, ou
dans lequel le sujet est dans la classe 0 ou R de RIFLE, et ladite étape de corrélation comprend l'assignation d'une probabilité que le sujet atteindra la classe I ou F de RIFLE dans les 48 heures, 36 heures, 24 heures, 18 heures, ou 12 heures, ou
dans lequel le sujet est dans la classe 0 ou R de RIFLE, et ladite étape de corrélation comprend l'assignation d'une probabilité que le sujet atteindra la classe F de RIFLE dans les 48 heures, 36 heures, 24 heures, 18 heures, ou 12 heures, ou
dans lequel le sujet est dans la classe R de RIFLE, et ladite étape de corrélation comprend l'assignation d'une probabilité que le sujet atteindra la classe I ou F de RIFLE dans les 48 heures, 36 heures, 24 heures, 18 heures, ou 12 heures, ou
dans lequel le sujet est dans la classe R de RIFLE, et ladite étape de corrélation comprend l'assignation d'une probabilité que le sujet atteindra la classe F de RIFLE dans les 48 heures, 36 heures, 24 heures, 18 heures, ou 12 heures.

9. Procédé selon l'une des revendications 1 à 6, dans lequel le sujet est dans la classe 0, R ou I de RIFLE, et ladite étape de corrélation comprend l'assignation d'une probabilité que le sujet atteindra la classe F de RIFLE dans les 48 heures, 36 heures, 24 heures, 18 heures, ou 12 heures,
dans lequel le sujet est préférablement dans la classe I de RIFLE, et ladite étape de corrélation comprend l'assignation d'une probabilité que le sujet atteindra la classe F de RIFLE dans les 48 heures, 36 heures, 24 heures, 18 heures, ou 12 heures, ou
dans lequel le sujet n'est pas en insuffisance rénale aiguë, ou
dans lequel le sujet n'a pas connu d'augmentation de 1,5 fois ou plus de la créatinine sérique par rapport à une valeur de référence déterminée avant le moment où l'échantillon de fluide corporel est obtenu, ou
dans lequel le sujet a une production d'urine d'au moins 0,5 ml/kg/h au cours des 6 heures précédant le moment où l'échantillon de fluide corporel est obtenu, ou
dans lequel le sujet n'a pas connu d'augmentation de 0,3 mg/dl ou plus de la créatinine sérique par rapport à une valeur de référence déterminée avant le moment où l'échantillon de fluide corporel est obtenu, ou
dans lequel le sujet (i) n'a pas connu d'augmentation de 1,5 fois ou plus de la créatinine sérique par rapport à une valeur de référence déterminée avant le moment où l'échantillon de fluide corporel est obtenu, (ii) a une production d'urine d'au moins 0,5 ml/kg/h au cours des 6 heures précédant le moment où l'échantillon de fluide corporel est obtenu, et (iii) n'a pas connu d'augmentation de 0,3 mg/dl ou plus de la créatinine sérique par rapport à une valeur de référence déterminée avant le moment où l'échantillon de fluide corporel est obtenu.

10. Procédé selon l'une des revendications 1 à 6,
dans lequel ladite étape de corrélation comprend l'assignation d'un ou plusieurs parmi : une probabilité que dans les 48 heures le sujet (i) connaîtra une augmentation de 1,5 fois ou plus de la créatinine sérique, (ii) aura une production d'urine de moins de 0,5 ml/kg/h sur une période de 6 heures, ou (iii) connaîtra une augmentation de 0,3 mg/dl ou plus de la créatinine sérique,
dans lequel ladite étape de corrélation comprend préférablement l'assignation d'un ou plusieurs parmi : une probabilité que dans les 24 heures le sujet (i) connaîtra une augmentation de 1,5 fois ou plus de la créatinine sérique, (ii) aura une production d'urine inférieure à 0,5 ml/kg/h sur une période de 6 heures, ou (iii) connaîtra une augmentation de 0,3 mg/dl ou plus de la créatinine sérique,
dans lequel ladite étape de corrélation comprend préférablement l'assignation d'un ou plusieurs parmi : une probabilité que dans les 18 heures le sujet (i) connaîtra une augmentation de 1,5 fois ou plus de la créatinine sérique, (ii) aura une production d'urine de moins de 0,5 ml/kg/h sur une période de 6 heures, ou (iii) connaîtra une augmentation de 0,3 mg/dl ou plus de la créatinine sérique.

11. Procédé selon l'une des revendications 1 à 6, dans lequel le sujet n'a pas connu d'augmentation de 2 fois ou plus de la créatinine sérique par rapport à une valeur de référence déterminée avant le moment où l'échantillon de fluide corporel est obtenu, ou
dans lequel le sujet a une production d'urine d'au moins 0,5 ml/kg/h au cours des 12 heures précédant le moment où l'échantillon de fluide corporel est obtenu, ou
dans lequel ladite étape de corrélation comprend préférablement l'assignation d'une probabilité que dans les 48 heures, le sujet connaîtra une augmentation de 2 fois ou plus de la créatinine sérique,
dans lequel ladite étape de corrélation comprend préférablement l'assignation d'une probabilité que dans les 24 heures, le sujet connaîtra une augmentation de 2 fois ou plus de la créatinine sérique, ou
dans lequel ladite étape de corrélation comprend préférablement l'assignation d'une probabilité que dans les 18 heures, le sujet connaîtra une augmentation de 2 fois ou plus de la créatinine sérique.

12. Procédé selon l'une des revendications 1 à 6,
dans lequel ladite étape de corrélation comprend l'assignation d'un ou plusieurs parmi : une probabilité que dans les 48 heures, le sujet (i) connaîtra une augmentation de 3 fois ou plus de la créatinine sérique, ou (ii) aura une production d'urine inférieure à 0,3 ml/kg/h sur une période de 24 heures, ou une anurie sur une période de 12 heures, dans lequel ladite étape de corrélation comprend préférablement l'assignation d'un ou plusieurs parmi : une probabilité que dans les 24 heures, le sujet (i) connaîtra une augmentation de 3 fois ou plus de la créatinine sérique, ou (ii) aura une production d'urine inférieure à 0,3 ml/kg/h sur une période de 24 heures, ou une anurie sur une période de 12 heures,

dans lequel ladite étape de corrélation comprend préférablement l'assignation d'un ou plusieurs parmi : une probabilité que dans les 18 heures, le sujet (i) connaîtra une augmentation de 3 fois ou plus de la créatinine sérique, ou (ii) aura une production d'urine inférieure à 0,3 ml/kg/h sur une période de 24 heures, ou une anurie sur une période de 12 heures.

13. Procédé d'évaluation de l'état rénal chez un sujet, comprenant :

la réalisation d'une analyse qui produit un signal détectable indiquant le niveau d'une concentration physiologiquement pertinente de protéine 1 similaire à la chitinase-3 sur un échantillon de fluide corporel obtenu sur le sujet pour fournir un résultat d'analyse ; et

la corrélation du résultat d'analyse à l'état rénal du sujet, ladite étape de corrélation comprenant une corrélation du résultat d'analyse à une probabilité d'un ou plusieurs changements futurs de l'état rénal, le changement futur étant une amélioration future de la fonction rénale, et

dans lequel la corrélation comprend une comparaison d'un résultat d'analyse sous la forme de la concentration de protéine 1 similaire à la chitinase-3 à un seuil prédéterminé sélectionné pour indiquer la probabilité d'amélioration future de la fonction rénale,

dans lequel une probabilité accrue d'amélioration future de la fonction rénale est assignée au sujet lorsque la concentration mesurée est inférieure au seuil, par rapport à une probabilité assignée lorsque la concentration mesurée est supérieure au seuil.

14. Procédé selon l'une des revendications 1 à 13, dans lequel l'échantillon de fluide corporel est un échantillon d'urine.

15. Procédé selon la revendication 13, dans lequel ladite étape de corrélation comprend le fait d'évaluer si la fonction rénale s'améliore ou non chez un sujet qui a souffert d'une lésion de la fonction rénale, d'une fonction rénale réduite ou d'une IRA sur la base du résultat d'analyse.

16. Procédé selon la revendication 13, dans lequel une pluralité de résultats d'analyse sont combinés en utilisant une fonction qui convertit la pluralité de résultats d'analyse en un seul résultat composite.

17. Utilisation d'une concentration mesurée de protéine 1 similaire à la chitinase-3 sur un échantillon de fluide corporel obtenu sur un sujet pour l'assignation d'une probabilité d'un ou plusieurs changements futurs de l'état rénal au sujet, sélectionnés dans le groupe constitué d'une amélioration future de la fonction rénale et d'une insuffisance rénale aiguë (IRA) future, l'utilisation comprenant la comparaison d'une concentration mesurée de protéine 1 similaire à la chitinase-3 à une valeur seuil,

dans lequel une probabilité accrue d'amélioration future de la fonction rénale est assignée au sujet lorsque la concentration mesurée est inférieure au seuil, par rapport à une probabilité assignée lorsque la concentration mesurée est supérieure au seuil, ou

dans lequel une probabilité accrue d'évolution jusqu'à une IRA est assignée au sujet lorsque la concentration mesurée est supérieure au seuil, par rapport à une probabilité assignée lorsque la concentration mesurée est inférieure au seuil,

et dans lequel la probabilité d'IRA future est plus ou moins élevée dans les 48 heures suivant le moment où l'échantillon de fluide corporel est obtenu sur le sujet.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

### Non-patent literature cited in the description

- Harrison's Principles of Internal Medicine. McGraw Hill, 1741-1830
- Current Medical Diagnosis & Treatment. McGraw Hill, 2008, 785-815
- Merck Manual
- PRAUGHT ; SHLIPAK. *Curr Opin Nephrol Hypertens,* 2005, vol. 14, 265-270
- CHERTOW et al. *JAm Soc Nephrol,* 2005, vol. 16, 3365-3370
- LASSNIGG. *J Am Soc Nephrol,* 2004, vol. 15, 1597-1605
- BELLOMO et al. *Crit Care.,* 2004, vol. 8 (4), R204-12
- KELLUM. *Crit. Care Med.,* 2008, vol. 36, 141-45
- RICCI et al. *idney Int.,* 2008, vol. 73, 538-546
- MEHTA et al. *Crit. Care,* 2007, vol. 11, R31
- MCCOLLOUGH et al. *Rev Cardiovasc Med.,* 2006, vol. 7 (4), 177-197
- MADDENS et al. Chitinase-like Proteins are Candidate Biomarkers for Sepsis-induced Acute Kidney Injury. *Mol Cell Proteomics,* 2012, vol. 11 (6), 1-13
- BERNSTEN et al. Therapeutic dendritic cell vaccination of patients with metastatic renal cell carcinoma - A clinical, phase ½ trial. *Journal of Immunotherapy,* 2008, vol. 31 (8), 771-780
- JOHANSEN. Studies on serum YKL-40 as a biomarker in diseases with inflammation, tissue remodelling, fibroses and cancer. *Danish Medical Bulletin,* 2006, vol. 53 (2), 172-209
- THAKAR et al. *J. Am. Soc. Nephrol.,* 2005, vol. 16, 162-68
- MEHRAN et al. *J. Am. Coll. Cardiol.,* 2004, vol. 44, 1393-99
- WIJEYSUNDERA et al. *JAMA,* 2007, vol. 297, 1801-9
- GOLDSTEIN ; CHAWLA. *Clin. J. Am. Soc. Nephrol.,* 2010, vol. 5, 943-49
- CHAWLA et al. *Kidney Intl.,* 2005, vol. 68, 2274-80
- Fundamental Immunology. Raven Press, 1993
- WILSON. *J. Immunol. Methods,* 1994, vol. 175, 267-273
- YARMUSH. *J. Biochem. Biophys. Methods,* 1992, vol. 25, 85-97
- WARD et al. *Nature,* 1989, vol. 341, 544-546
- VAN ERP et al. *J. Immunoassay,* 1991, vol. 12, 425-43
- NELSON ; GRISWOLD. *Comput. Methods Programs Biomed.,* 1988, vol. 27, 65-8
- CWIRLA et al. *Proc. Natl. Acad. Sci. USA,* 1990, vol. 87, 6378-82
- DEVLIN et al. *Science,* 1990, vol. 249, 404-6
- SCOTT ; SMITH. *Science,* 1990, vol. 249, 386-88
- FISCHER et al. *Intensive Care Med.,* 2003, vol. 29, 1043-51
- BAGSHAW et al. *Nephrol. Dial. Transplant.,* 2008, vol. 23, 1203-1210
- Merck Manual of Diagnosis and Therapy. Merck Research Laboratories, 1999
- HANLEY, J. A. ; MCNEIL, B.J. The meaning and use of the area under a receiver operating characteristic (ROC) curve. *Radiology,* 1982, vol. 143, 29-36